(19) Europäisches Patentamt
European Patent Office
Office européen des brevets

(11) Numéro de publication : 0 495 905 B1

# (12) FASCICULE DE BREVET EUROPEEN

(45) Date de publication du fascicule du brevet : **02.02.94 Bulletin 94/05**

(51) Int. Cl.[5] : **C07D 303/36,** C07D 303/02, C08F 2/38

(21) Numéro de dépôt : **90916161.4**

(22) Date de dépôt : **18.10.90**

(86) Numéro de dépôt international : **PCT/FR90/00754**

(87) Numéro de publication internationale : **WO 91/05779 02.05.91 Gazette 91/10**

## (54) DISULFURES DE THIURAME A GROUPEMENTS EPOXY, LEUR PREPARATION ET LEUR UTILISATION DANS LA POLYMERISATION.

(30) Priorité : **19.10.89 FR 8913688**

(43) Date de publication de la demande : **29.07.92 Bulletin 92/31**

(45) Mention de la délivrance du brevet : **02.02.94 Bulletin 94/05**

(84) Etats contractants désignés : **AT BE CH DE DK ES FR GB GR IT LI LU NL SE**

(56) Documents cités :
**EP-A- 161 502**
**EP-A- 237 792**

(73) Titulaire : **CRAY VALLEY SA**
**Tour Total, 24 cours Michelet**
**F-92800 Puteaux (FR)**

(72) Inventeur : **CLOUET, Gilbert**
**6, rue des Jardins**
**F-67610 La Wantzenau (FR)**

(74) Mandataire : **Chaillot, Geneviève**
**Cabinet CHAILLOT 21, avenue Louise de Bettignies**
**F-92700 Colombes (FR)**

EP 0 495 905 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention porte sur de nouveaux disulfures de thiurame à groupements époxy, sur un procédé de fabrication de ces disulfures, et sur leur utilisation comme agents ayant la triple fonction d'initiateur, d'agent de transfert de chaîne et d'agent de terminaison (désignés par l'abréviation "iniferters"), dans la polymérisation radicalaire de monomères vinyliques et diéniques conduisant à des oligomères et polymères vinyliques et diéniques à fonctions terminales époxy.

L'invention porte également sur ces oligomères et polymères vinyliques et diéniques qui sont utiles pour fabriquer des copolymères séquencés par condensation de leurs fonctions terminales époxy avec au moins un autre oligomère ou polymère fonctionnel.

Des disulfures de thiurame fonctionnels ont déjà été proposés dans la demande de brevet européen EP-A-0237792 à titre d'iniferters, les groupes fonctionnels envisagés comprenant les fonctions hydroxyle, carboxyle et amine.

La demande de brevet européen EP-A-0161502 décrit des (co)polymères linéaires ayant une masse moléculaire moyenne d'environ 1500-15 000, et obtenus par (co)polymérisation en solution de monomères à insaturation éthylénique, dans un milieu de solvant organique contenant un initiateur de radicaux libres et une proportion d'un agent de terminaison de chaîne de formule :

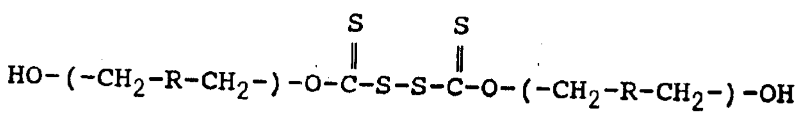

avec R = radical organique exempt de groupes réactifs.

La présente invention a donc d'abord pour objet des disulfures de thiurame fonctionnels de formule:

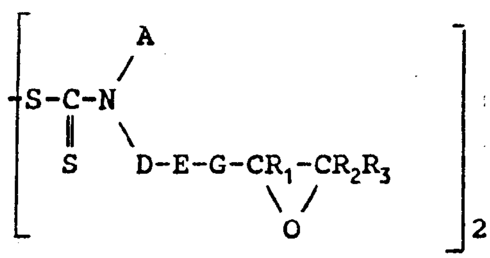
(Ia)

ou

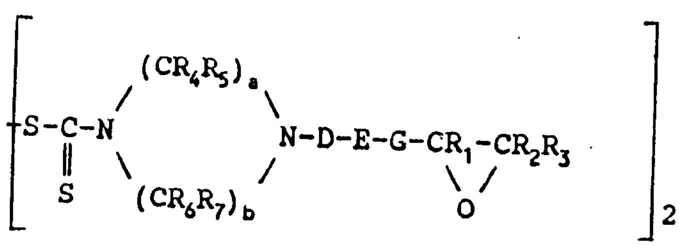
(Ib)

ou encore

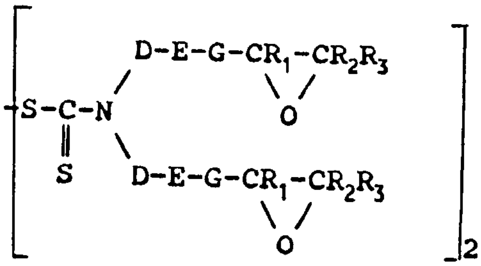

(Ic)

dans laquelle :

- A est choisi parmi les groupes alkyle, cycloalkyle, aryle, arylalkyle, alkylaryle, les séquences polyoxyalkylène représentées par les formules suivantes :

$$R_{10}\text{-O-(-}CH_2\text{-}CH_2\text{-O-)}_c\text{-}CH_2\text{-}CH_2\text{-}$$

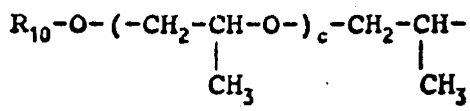

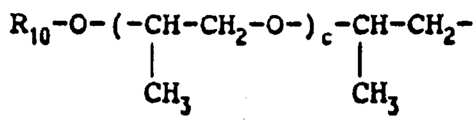

$$R_{10}\text{-O-[-(}CH_2)_4\text{-O-]}_c\text{-(}CH_2)_4\text{-}$$

où :

- $R_{10}$ représente un reste alkyle, cycloalkyle ou aryle ; et
- c vaut de 1 à 1000, de préférence de 1 à 200 ; et les séquences polyester représentées par les formules suivantes :

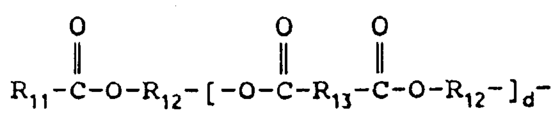

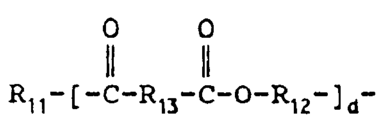 ;

et

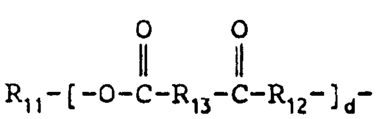

où :

- $R_{11}$ est tel que défini ci-dessus pour $R_{10}$ ;

- $R_{12}$ et $R_{13}$ sont choisis parmi les groupes alkylène, cycloalkylène, arylène, alcénylène, alcénylarylène et peuvent comprendre au moins un atome de nature différente au sein de la chaîne et/ou du cycle et/ou au moins un reste

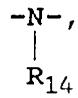,

$R_{14}$ étant choisi parmi les groupes alkyle, cycloalkyle et aryle, et pouvant comporter des substituants ; et

- d vaut de 1 à 300, de préférence, de 1 à 50 ;

- D et G sont choisis parmi les groupes alkylène, cycloalkylène, arylène, alkylarylène et arylalkylène et peuvent comporter au moins un atome de nature différente au sein de la chaîne et/ou du cycle,
- E représente :
  . un reste

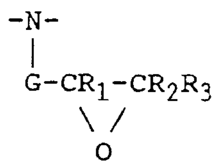,

ou bien

. un reste -J-K-, J étant choisi parmi les groupements $CR_8R_9O$,

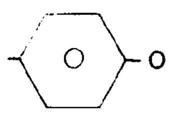 et 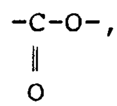,

et

K représentant une simple liaison ou, dans le cas des composés (Ia), J peut représenter l'atome d'oxygène et K peut représenter un reste

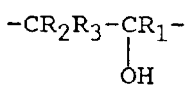 (a), ou 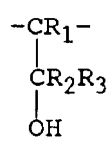 (b),

- $R_1$, $R_2$, $R_3$, $R_8$ et $R_9$ sont choisis parmi l'atome d'hydrogène et les restes alkyle, cycloalkyle, aryle, alkylaryle et arylalkyle,
- $R_4$, $R_5$, $R_6$ et $R_7$ sont choisis parmi l'atome d'hydrogène et les restes alkyle, et
- a et b sont choisis parmi les nombres entiers de 1 à 3.

On peut mentionner, comme exemples de groupes $R_{12}$ et $R_{13}$ insaturés, les groupes éthylène, butène-2, styrène, vinyltoluène, ainsi que ceux de formules :

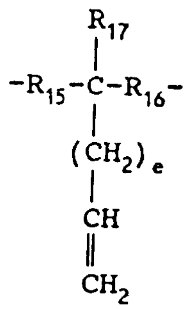 , 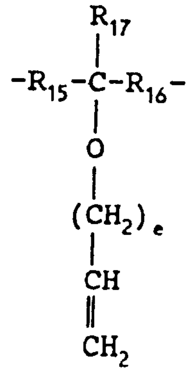 , 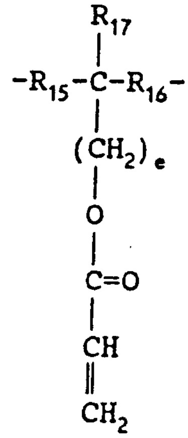

dans lesquelles :

- e est un nombre entier de 1 à 12 ;
- $R_{15}$ et $R_{16}$ représentent une simple liaison, ou bien un groupe alkylène, cycloalkylène ou arylène ; et
- $R_{17}$ est tel que défini ci-dessus pour $R_1$.

Les groupes alkyle et alkylène entrant respectivement dans les définitions ci-dessus sont notamment des groupes en $C_1$-$C_{12}$, en particulier en $C_1$-$C_6$ ; les groupes cycloalkyle et cycloalkylène sont notamment des groupes mono- ou polycycliques en $C_3$-$C_{12}$, en particulier en $C_5$-$C_7$ ; les groupes aryle et arylène sont notamment des groupes en $C_6$-$C_{15}$ incluant un ou plusieurs noyaux aromatiques tels que phényle, biphényle ou naphtyle.

Les composés de formule (Ia) ou (Ib) ou (Ic) dans laquelle E représente un reste

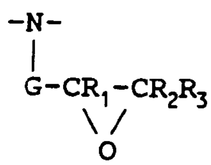

peuvent être fabriqués par un procédé caractérisé en ce que :

- dans une première étape, on fait réagir une amine primaire de formule :

$$A\text{-}NH\text{-}D\text{-}NH_2 \qquad (IIa)$$

ou

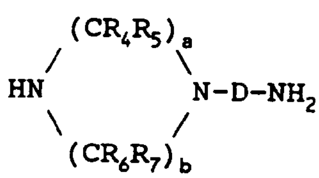 (IIb)

ou $H_2N\text{-}D\text{-}NH\text{-}D\text{-}NH_2$ (IIc)

avec un composé carbonylé de formule :

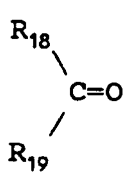 (III)

dans laquelle $R_{18}$ et $R_{19}$ sont tels que définis ci-dessus pour $R_1$, avec la condition qu'ils ne sont pas tous deux l'atome d'hydrogène, pour obtenir le composé de formule :

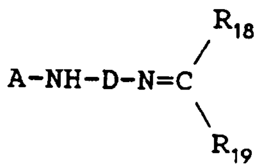

(IVa)

ou

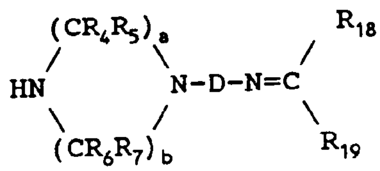

(IVb)

ou

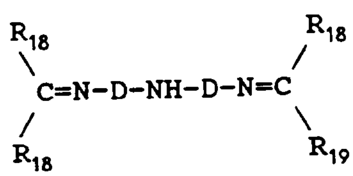

(IVc),

- dans une seconde étape, on fait réagir le composé (IVa) ou (IVb) ou (IVc) avec le disulfure de carbone en présence d'au moins un agent oxydant, pour obtenir le composé :

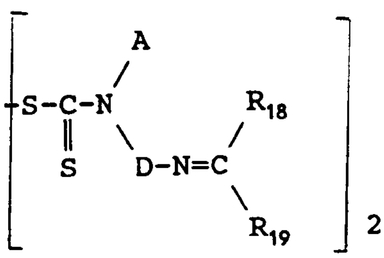

(Va)

ou

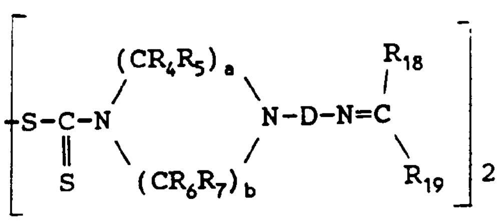

(Vb)

ou

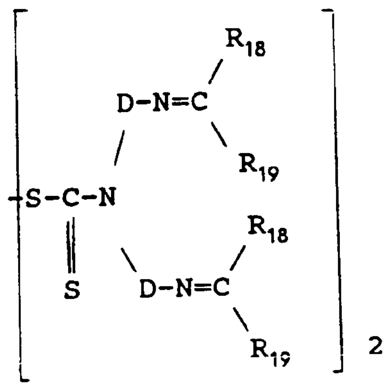

(Vc),

- dans une troisième étape, on hydrolyse ou on réduit le composé (Va) ou (Vb) ou (Vc) pour obtenir le composé de formule :

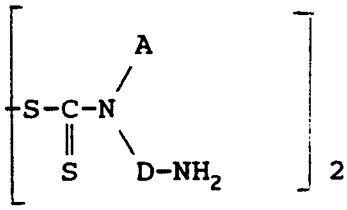

(VIa)

ou

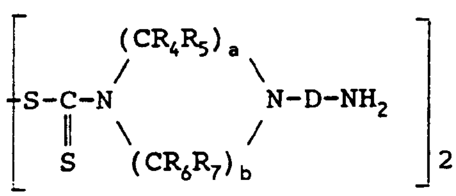

(VIb)

ou

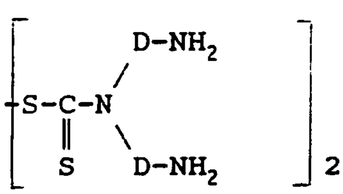

(VIc),

puis

- dans une quatrième étape, on fait réagir le composé (VIa) ou (VIb) ou (VIc) en milieu basique, avec un époxyde de formule :

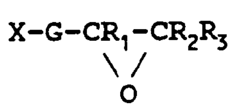

(VII)

dans laquelle X représente un atome d'halogène, le rapport molaire du composé (VII) au composé (VIa) ou (VIb) ou (VIc) étant au moins égal à 2.

Les conditions expérimentales de la première étape dépendent de la nature de l'amine et du composé carbonylé (III). Habituellement, il est souhaitable d'éliminer l'eau au fur et à mesure qu'elle se forme, par distillation ou par utilisation d'un solvant formant un azéotrope, comme le toluène. On peut aussi utiliser un alcool, comme l'éthanol, ou un mélange eau-alcool. L'élimination de l'eau est nécessaire avec les diaryl- et les arylalkylcétones. Les aldéhydes aromatiques réagissent à des températures relativement basses, avec ou sans solvant. Avec les cétones, en particulier avec les cétones aromatiques, des températures supérieures, des temps de réaction plus longs, et un catalyseur sont habituellement requis, en plus de l'élimination de l'eau au fur et à mesure qu'elle se forme. La réaction peut être catalysée par les acides. La température de réaction est généralement comprise entre environ 0°C et environ 40°C.

Comme exemples d'amines primaires de formule (IIa) on peut citer notamment le 1-amino 2-aminométhyl 3,3,5-triméthylcyclopentane, le 1-amino 2-aminométhyl 3,5,5-triméthylcyclopentane, la triacétonediamine et les diamines de formule A-NH-$(CH_2)_3NH_2$, dans laquelle A est un radical oléyle, arachidyle ou béhényle, commercialisées par la société CECA sous la marque DINORAM.

A la seconde étape, on peut utiliser un rapport sensiblement équimolaire du composé (IVa) ou (IVb) ou (IVc) à $CS_2$, la réaction pouvant être effectuée en présence d'une amine tertiaire (par exemple, la triéthylamine ou la pyridine), par exemple à raison d'environ 1 mole par mole du composé (IVa) ou (IVb) ou (IVc). L'agent oxydant est ajouté, par exemple, à raison d'environ 1 mole par mole de $CS_2$. Il peut être choisi, entre autres, parmi l'iode, l'eau oxygenée, les hypochlorites de métaux alcalins, les hydroperoxydes d'alkyle et d'aryle, et l'hexacyanoferrate de potassium. La réaction peut être conduite en milieu solvant, par exemple, dans l'eau, un alcool ou un mélange l'eau oxygénée, les hypochlorites de métaux alcalins, les hydroperoxydes d'alkyle et d'aryle, et l'hexacyanoferrate de potassium. La réaction peut être conduite en milieu solvant, par exemple, dans l'eau, un alcool ou un mélange eau-alcool, $CS_2$ ou l'amine tertiaire pouvant du reste faire office de solvant. La réaction est conduite généralement à une température se situant entre environ -20°C et +80°C et, de préférence, entre +15°C et +40°C environ.

L'hydrolyse de la troisième étape est généralement conduite en présence d'eau acidulée, par exemple un mélange 50/50 acide chlorhydrique/eau, à une température pouvant être comprise entre environ 20°C et 120°C, sous agitation énergique. Le produit résultant est isolé, lavé à l'eau ou au méthanol, puis séché sous vide, par exemple, à environ 30°C.

La fonction imine du composé (Va) ou (Vb) ou (Vc) peut également être réduite au cours de la troisième étape à l'aide d'un hydrure, comme $AlLiH_4$, $NaBH_4$, etc., à une température généralement comprise entre environ -20°C et 30°C, de préférence entre environ 5°C et 10°C.

La réaction de la quatrième étape est de préférence conduite en présence d'hydroxyde alcalin (sodium, potassium ou lithium), à une température ne dépassant généralement pas 90°C, soigneusement contrôlée pour éviter des réactions secondaires. L'épichlorhydrine constitue l'exemple le plus simple de composé (VII).

Le produit (Ia) ou (Ib) ou (Ic) obtenu est par exemple dissous dans le toluène, lavé à l'eau, puis filtré ; la couche toluénique est recueillie, de laquelle on chasse le toluène et le composé (VII) résiduel par exemple par distillation sous pression réduite.

Les composés de formule (Ia) ou (Ib) ou (Ic) dans laquelle E représente -J-K-, K représentant une simple liaison, peuvent être préparés par un procédé caractérisé en ce que :

- dans une première étape, on fait réagir un composé de formule :

A-NH-D-JH (VIIIa)

ou

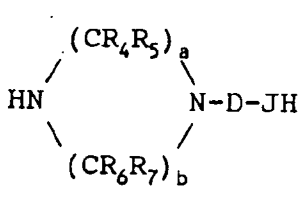

(VIIIb)

ou HJ-D-NH-D-JH (VIIIc)

ou bien l'un de ses sels d'ammonium quaternaire avec le disulfure de carbone, en présence d'au moins un agent oxydant, pour obtenir le composé :

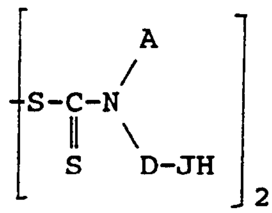 (IXa)

ou

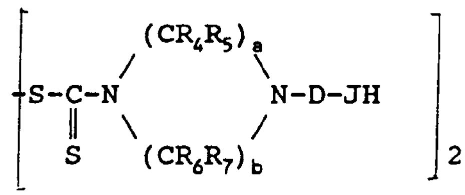 (IXb)

ou

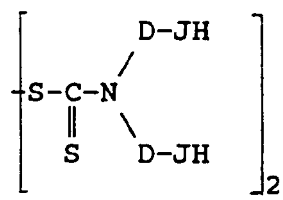 (IXc)

- puis, dans une seconde étape, on fait réagir le composé (IXa) ou (IXb) ou (IXc) en milieu basique avec un excès molaire de l'époxyde de formule (VII).

A la première étape, on peut utiliser un rapport sensiblement équimolaire du composé (VIIIa) ou (VIIIb) ou (VIIIc) ou son sel à $CS_2$, la réaction pouvant être effectuée en présence d'une amine tertiaire (par exemple, pyridine ou triéthylamine), par exemple à raison d'environ 1 mole par mole du composé (VIIIa) ou (VIIIb) ou (VIIIc), ou d'environ 2 moles par mole du sel correspondant. Les autres conditions de la réaction sont celles de la seconde étape du procédé décrit précédemment.

Comme exemples de composés de formule (VIIIa) on peut citer notamment le méthylaminoéthanol, l'éthylaminoéthanol, le n-butylaminoéthanol, le tertiobutylaminoéthanol, l'isopropylaminoéthanol, la N-propyléthanolamine, l'hexylaminoéthanol, l'amylaminoéthanol et le triacétone aminoalcool. Comme exemples de composés de formule (VIIIc) on peut citer notamment la diisopropanolamine et le 1-(2-hydroxyéthanolamino)-propanol-2.

La seconde étape peut, par exemple, être effectuée dans les conditions de la quatrième étape du procédé décrit précédemment.

Les composés de formule (Ia) ou (Ic) dans laquelle E représente -J-K, K représentant un reste

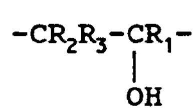 (a) ou 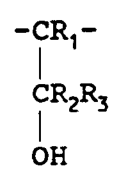 (b)

peuvent être préparés par un procédé caractérisé en ce que:

- dans une première étape, on fait réagir un composé de formule (VIIIa) ou (VIIIc) avec un haloacide HX, pour obtenir le sel de formule :

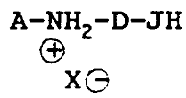

(Xa) ;

ou

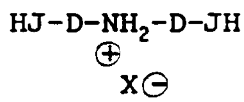

(Xc)

- dans une seconde étape, on fait réagir le sel (Xa) ou (Xc) en milieu basique avec un diépoxyde de formule :

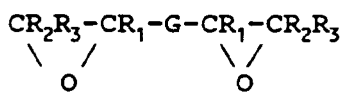

(XI)

pour obtenir un mélange statistique des composés de formules :

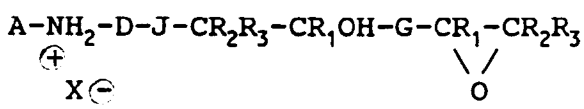

(XIIa)

et

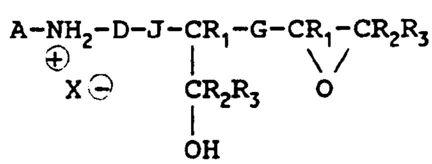

(XIIIa) ;

ou bien

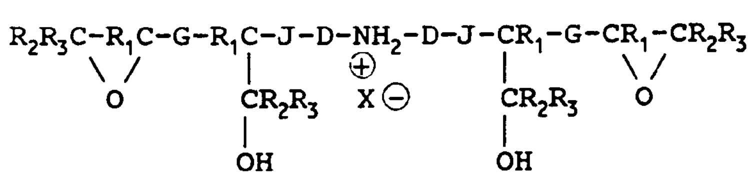

(XIIIc) ;

et

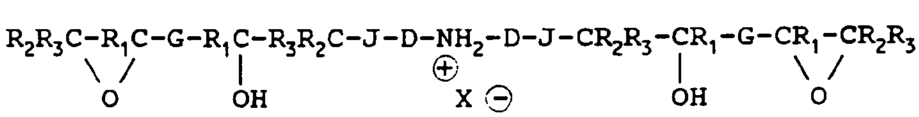

(XIIc) ;

- dans une troisième étape, on fait réagir les composés (XIIa) et (XIIIa) ou bien (XIIc) et (XIIIc) avec le

disulfure de carbone, en présence d'au moins un agent oxydant.

La première étape est généralement conduite à une température comprise entre environ -20°C et 40°C, par exemple à 20°C.

A la seconde étape, on utilise généralement un très large excès molaire du composé (XI) par rapport au composé (Xa) ou (Xc), et on travaille de préférence en l'absence d'amine tertiaire. La réaction est de préférence catalysée par un acide ($H_2SO_4$, $SnCl_4$) et effectuée à une température d'environ 50 à 90°C, pendant environ 1/2 heure à 3 heures. Comme exemples de diépoxydes de formule (XI), on peut citer notamment le 1,2,3,4-diépoxybutane, le 1,2,5,6-diépoxycyclooctane, le 1,2,7,8-diépoxyoctane, le diglycidyl éther du bisphénol A, le diglycidyl éther du bisphénol F et le tétraglycidyldiaminodiphénylméthane.

Pour la troisième étape, on peut utiliser les conditions de la seconde étape du premier procédé décrit précédemment.

Les composés de formule (Ia) ou (Ic) dans laquelle J représente l'atome d'oxygène et K représente une simple liaison ou bien l'un des restes (a) et (b) définis plus haut peuvent être préparés par un procédé caractérisé en ce que l'on fait réagir un disulfure de thiurame dihydroxylé, diaminé, tétrahydroxylé ou tétraaminé déjà connu par le document EP-A-237 792, de formule

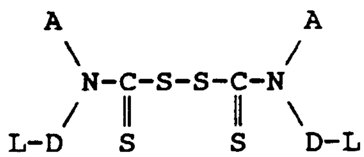

(XVa),

ou

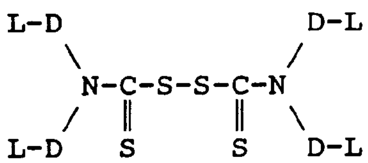

(XVc),

L désignant un groupe hydroxyle ou bien $NHR_{20}$ dans lequel $R_{20}$ est tel que défini pour $R_4$, avec un époxyde de formule (VII) ou bien un diépoxyde de formule (XI), puis en procédant le cas échéant à l'élution du milieu réactionnel sur une colonne d'alumine basique afin de régénérer la fonction époxy des coproduits hydroxylés éventuellement formés.

La présente invention a également pour objet l'utilisation des disulfures de thiurame fonctionnels de formule (Ia) ou (Ib) ou (Ic) comme agent iniferter dans la polymérisation radicalaire de monomères vinyliques Mv, ledit agent iniferter étant de préférence introduit en début de polymérisation avec le mélange des monomères.

Comme monomères vinyliques Mv, on peut citer notamment :

- un acrylate ou méthacrylate d'alkyle dont le groupe alkyle linéaire ou ramifié, et comprenant le cas échéant au moins un hétéroatome, sous forme par exemple d'atome d'halogène, de groupe carboxyle ou amino, possède de 1 à 20 atomes de carbone, tels que ceux de méthyle, éthyle, n-butyle, tertiobutyle, 2-éthylhexyle, stéaryle, 2,2,2-trifluoroéthyle, hydroxyéthylimidazolidone et hydroxyéthyloxazolidone,
- un acrylate ou méthacrylate d'aryle tel que les méthacrylates de benzyle et de phényle,
- un hydrocarbure vinylaromatique tel que le styrène, le vinyltoluène, l'$\alpha$-méthylstyrène, le méthyl-4 styrène, le méthyl-3 styrène, le méthoxy-4 styrène, l'hydroxyméthyl-2 styrène, l'éthyl-4 styrène, l'éthoxy-4 styrène, le diméthyl-3,4 styrène, le chloro-2 styrène, le chloro-3 styrène, le chloro-4 méthyl-3 styrène, le tert.-butyl-3 styrène, le dichloro-2,4 styrène, le dichloro-2,6 styrène et le vinyl-l naphtalène,
- un acrylate ou méthacrylate époxydé tel que l'acrylate et le méthacrylate de glycidyle, le 2-époxyéthylbicyclo[2.2.1]hept-5(6)-yl (méth)acrylate et le produit d'époxydation de l'acrylate de dicyclopentényloxyéthyle,
- un acrylamide ou méthacrylamide, un acrylate ou méthacrylate de dialkylaminoalkyle et leurs sels quaternaires,
- l'acrylate et le méthacrylate de (norbornyloxy-2)-2 éthyle et de (diméthanodécahydronaphtyloxy-2)-2 éthyle,

- un nitrile insaturé tel que l'acrylonitrile ou le méthacrylonitrile,
- une maléimide N-substituée telle que la N-éthylmaléimide, la N-isopropylmaléimide, la N-n-butylmaléimide, la N-isobutylmaléimide, la N-terbutylmaléimide, la N-n-octylmaléimide, la N-cyclohexylmaléimide, la N-benzylmaléimide et la N-phénylmaléimide,
- un diène tel que le 1,3-butadiène, l'isoprène, le 1,3-pentadiène, le 1,4-pentadiène, le 1,4-hexadiène, le 1,5-hexadiène, le 1,9-décadiène, le 5-méthylène- 2-norbornène, le 5-vinyl-2-norbornène, les 2-alkyl-2,5-norbornadiènes, le 5-éthylidène-2-norbornène, le 5-(2-propényl)-2-norbornène, le 5-(5-hexényl)-2-norbornène, le 1,5-cyclooctadiène, le bicyclo [2,2,2] octa-2,5-diène, le cyclopentadiène, le 4,7,8,9-tétrahydroindène et l'isopropylidène tétrahydroindène,
- un halogénure de vinyle ou de vinylidène, tel que le bromure de vinyle et les chlorures de vinyle et de vinylidène,
- les esters vinyliques tels que l'acétate de vinyle et le crotonate de vinyle ;
- le 9-vinylcarbazole, la vinyl-2-pyridine, la vinyl-4-pyridine, la 1-vinyl-2-pyrrolidinone, le vinyltriméthoxysilane, le vinyltriméthylsilane et le 1-vinylimidazole, et
- les méthacrylates de trialkylsilyle ou de trialcoxysilyle tels que le méthacrylate de 3-(triméthoxysilyl) propyle, le méthacrylate de triméthylsilyle et le méthacrylate de 2-(triméthylsilyloxy)éthyle.

A titre d'exemple pour un composé (Ia) ou (Ic) et pour un monomère Mv, la polymérisation selon l'invention conduit à la formation d'un oligomère ou polymère $\alpha,\omega$-di-fonctionnel de formule :

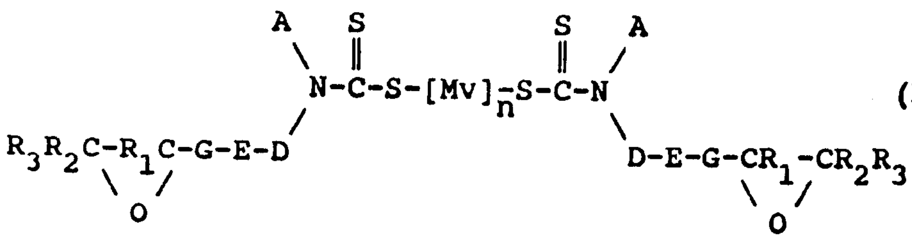
(XIVa),

ou

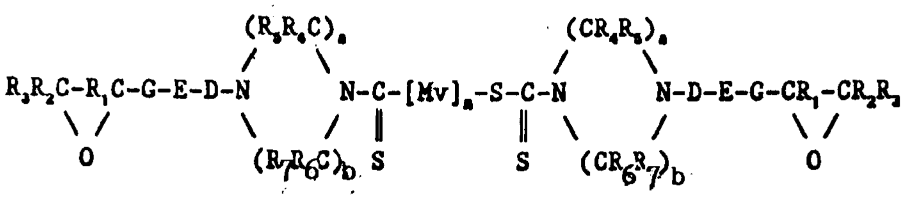
(XIVb),

ou

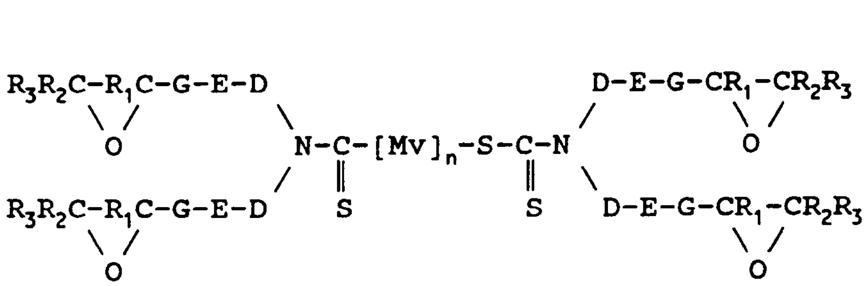
(XIVc)

dans laquelle n est un nombre entier tel que la masse moléculaire moyenne en nombre de la séquence dérivée de Mv soit comprise entre $10^2$ et $10^6$ environ, et plus particulièrement entre $10^3$ et $10^5$ environ.

Les conditions de polymérisation en présence de l'iniferter sont naturellement variables selon la nature du

monomère vinylique Mv mais comprennent généralement une température comprise entre 50°C et 130°C environ et une durée comprise entre 0,5 et 48 heures environ.

La quantité d'iniferter introduite est généralement comprise entre environ 5 x $10^{-5}$ mole/l et 0,1 mole/l par rapport au monomère vinylique.

L'invention porte également sur les oligomères et polymères vinyliques à terminaisons époxy obtenus, tels que définis ci-dessus.

De tels oligomères et polymères vinyliques à terminaison époxy peuvent aussi être obtenus en polymérisant d'abord le monomère vinylique Mv en présence d'un disulfure de thiurame hydroxylé ou aminé de formule (XVa) ou (XVc) puis en faisant réagir le polymère vinylique à terminaison hydroxyle ou amine obtenu avec un époxyde de formule (VII) ou bien un diépoxyde de formule (XI). Une autre méthode de synthèse consiste aussi à polymériser d'abord le monomère vinylique Mv en présence d'un disulfure de thiurame fonctionnalisé de formule (VIa), (VIb), (VIc), (IXa), (IXb) ou (IXc) puis à faire réagir le polymère vinylique fonctionnalisé ainsi obtenu avec un époxyde de formule (VII).

Les exemples suivants sont donnés à titre illustratif et non limitatif de la présente invention.

Exemple 1

(a) Préparation du disulfure de bis-4-(2-benzylidèneaminoéthyl)-pipérazine thiurame

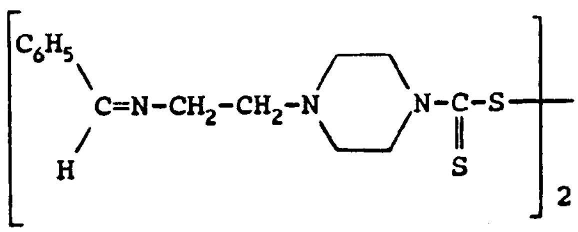

et, par réduction de ce dernier, du bis-4-(2-amino)pipérazine thiurame de formule :

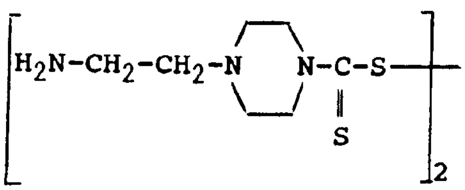

A un mélange de 60 ml d'eau, 60 ml d'éthanol et 25,8 g (0,2 mole) de 1-(2-aminoéthyl)pipérazine, on ajoute 21,2 g (0,22 mole) de benzaldéhyde. On porte ensuite le mélange à une température comprise entre 0 et 5°C. On y introduit goutte à goutte 15,2 g (0,2 mole) de $CS_2$. Un précipité apparaît, qui est redissous par l'introduction d'un mélange $H_2O/C_2H_5OH$ (200 ml/260 ml).

Après dissolution, on ajoute 27,8 ml (0,2 mole) de triéthylamine. On oxyde le milieu avec 66 g (0,2 mole) d'hexacyanoferrate de potassium III. Il en résulte une précipitation. On filtre la solution, on lave le précipité à l'eau puis au méthanol, et on le sèche sous vide à 30°C.

On obtient 53 g d'une substance contenant 57,1% C, 6,1% H, 14,4% N et 21,8% S.

On additionne 29,2 g (0,1 mole) de cette substance sur 124 g (0,4 mole) d'hydrure de lithium et d'aluminium en suspension dans du toluène maintenu entre 5 et 10.C. Puis on lave le mélange 3 fois de suite avec de l'eau, on sèche la phase organique sur $Na_2SO_4$. Après évaporation du toluène, on obtient un produit contenant 41,1% C, 6,8% H, 20,5% N et 31,3% S.

(b) Préparation du disulfure de bis 4-(N,N-diglycidyl 2-éthyl)-pipérazine 1-thiurame

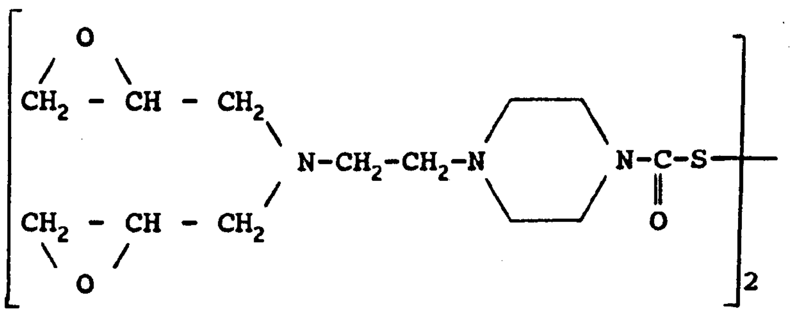

Dans un ballon équipé d'un agitateur, d'un réfrigérant et d'une ampoule à brome, on introduit 10,2 g (0,05 mole) du composé obtenu à l'étape (a) dans 100 ml de toluène, ainsi que 8 g de NaOH en solution aqueuse à 10%. On chauffe le mélange à 45°C. Par l'intermédiaire de l'ampoule à brome, on introduit 110 ml (92,4 g ; 1 mole) d'épichlorohydrine, de façon que la température ne dépasse pas 90°C. Après l'addition, on maintient le mélange à cette température pendant 90 minutes. On arrête l'agitation. Deux phases apparaissent. On sépare la solution aqueuse par décantation. On lave la phase organique avec de l'eau jusqu'à l'obtention d'un pH neutre des eaux de lavage.

On sèche la phase organique sur $Na_2SO_4$. Après évaporation du solvant et séchage sous vide à 30°C, on obtient une poudre contenant 49,3% C ; 6,9% H ; 13,3% N ; 20,2% S et 10,1% O.

Exemples 2 à 7

Polymérisation radicalaire du méthacrylate de méthyle (Exemples 2 à 4) et du styrène (Exemples 5 à 7) en présence de l'iniferter de l'Exemple 1.

La polymérisation radicalaire à l'aide des iniferters selon l'invention est effectuée, à l'abri de la lumière, dans des ballons munis d'une agitation, qui sont scellés sous vide ou maintenus sous gaz inertes ($N_2$, argon) après introduction des réactifs. Lorsque l'iniferter est soluble dans le monomère, la polymérisation peut être réalisée en masse ; dans le cas contraire, le monomère est dilué par moitié avec du toluène (ou tout autre solvant de l'iniferter et du monomère). La quantité désirée d'iniferter, le monomère et éventuellement le solvant sont introduits dans le ballon qui est ensuite dégazé. On conduit la polymérisation en immergeant le ballon dans un bain d'huile thermostaté à la température requise. Après polymérisation, le ballon est retiré, réfrigéré dans un mélange isopropanol-carboglace, et le polymère est dilué par du toluène, puis précipité goutte à goutte dans du méthanol (ou de l'heptane). Le précipité ou le produit résineux obtenu est recueilli dans un creuset en verre fritté, lavé à l'heptane, séché à 45°C pendant 12 heures méthanol (ou de l'heptane). Le précipité ou le produit résineux obtenu est recueilli dans un creuset en verre fritté, lavé à l'heptane, séché à 45°C pendant 12 heures sous vide.

On a effectué des polymérisations du méthacrylate de méthyle avec des concentrations initiales en iniferter de 0,09 à 0,05 mole/l, en faisant varier la température et le temps de polymérisation. De même on a effectué des polymérisations du styrène à 70°C en faisant varier la concentration initiale en iniferter et le temps de polymérisation. On a mesuré dans chaque cas la masse moléculaire moyenne en nombre du polymère obtenu et son indice de polydispersité $I = \overline{Mw}/\overline{Mn}$. L'équivalent époxy pour 100 g de produit a été déterminé par spectrométrie infrarouge à transformée de Fourier et également calculé à partir du % en poids de soufre trouvé dans le polymère.

| Ex. | $[In] \times 10^3$ | Température °C | Temps de polym en minutes | $\overline{Mn}$ | I | Equivalent époxy pour 100 g de produit |
|---|---|---|---|---|---|---|
| 2 | 50 | 95 | 720 | 26 000 | 2,5 | $1,5 \times 10^{-2}$ |
| 3 | 60 | 70 | 170 | 21 000 | 2,3 | $1,9 \times 10^{-2}$ |
| 4 | 90 | 85 | 900 | 10 000 | 1,7 | $4 \times 10^{-2}$ |
| 5 | 15 | 70 | 170 | 7 000 | 2,3 | $5,7 \times 10^{-2}$ |
| 6 | 25 | 70 | 900 | 5 000 | 2,1 | 0,08 |
| 7 | 30 | 70 | 900 | 4 000 | 2 | 0,01 |

[In] : concentration en iniferter en mole/l de monomère

$\overline{Mn}$ = masse moléculaire moyenne en nombre déterminée par chromatographie de perméation de gel.

## Revendications

**Revendications pour les Etats contractants suivants : AT, BE, CH, DE, DK, FR, GB, GR, IT, LI, LU, NL, SE**

1. Disulfures de thiurame fonctionnels de formule:

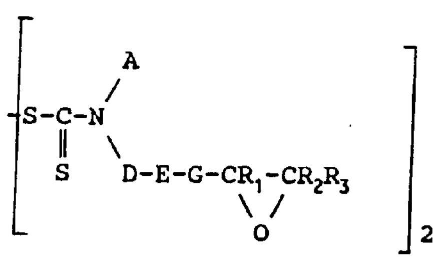

(Ia)

ou

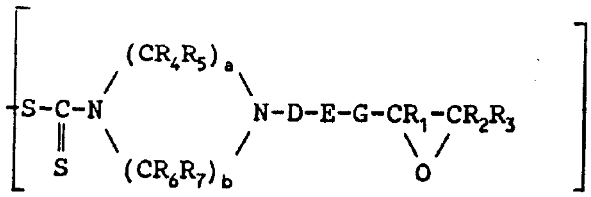

(Ib)

ou encore

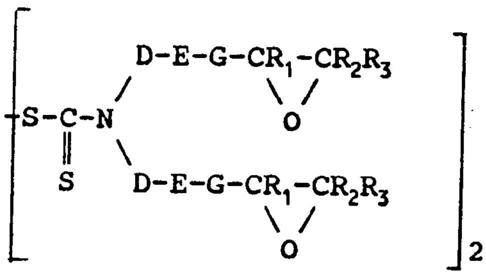

(Ic)

dans laquelle :

- A est choisi parmi les groupes alkyle, cycloalkyle, aryle, arylalkyle, alkylaryle, les séquences polyoxyalkylène représentées par les formules suivantes :

$$R_{10}\text{-O-(-}CH_2\text{-}CH_2\text{-O-)}_c\text{-}CH_2\text{-}CH_2\text{-}$$

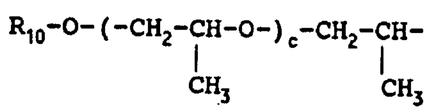

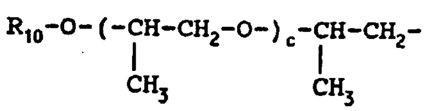

$$R_{10}\text{-O-[-}CH_2)_4\text{-O-]}_c\text{-(}CH_2)_4\text{-}$$

où :

- $R_{10}$ représente un reste alkyle, cycloalkyle ou aryle ; et
- c vaut de 1 à 1000 ;

et les séquences polyester représentées par les formules suivantes :

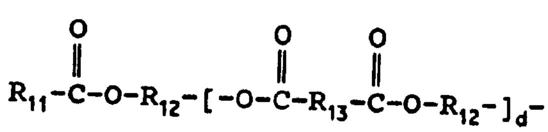

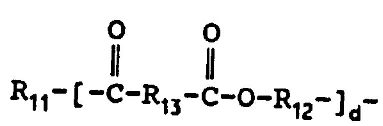 ;

et

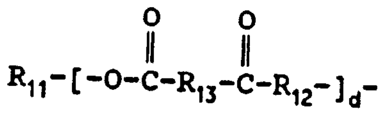

où :

- $R_{11}$ est tel que défini ci-dessus pour $R_{10}$ ;
- $R_{12}$ et $R_{13}$ sont choisis parmi les groupes alkylène, cycloalkylène, arylène, alcénylène, alcénylarylène et peuvent comprendre au moins un atome de nature différente au sein de la chaîne et/ou du cycle et/ou au moins un reste

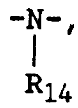

  $R_{14}$ étant choisi parmi les groupes alkyle, cycloalkyle et aryle, et pouvant comporter des substituants ; et
- d vaut de 1 à 300,
- D et G sont choisis parmi les groupes alkylène, cycloalkylène, arylène, alkylarylène et arylalkylène et peuvent comporter au moins un atome de nature différente au sein de la chaîne et/ou du cycle,
- E représente :
  . un reste

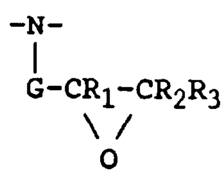

,

ou bien

. un reste -J-K-, J étant choisi parmi les groupements $CR_8R_9O$,

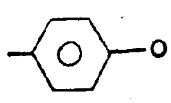

et

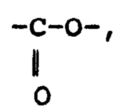

et

K représentant une simple liaison ou, dans le cas des composés (Ia), J peut représenter l'atome d'oxygène et K peut représenter un reste

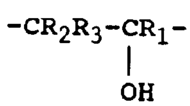

(a), ou

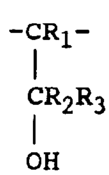

(b),

- $R_1$, $R_2$, $R_3$, $R_8$ et $R_9$ sont choisis parmi l'atome d'hydrogène et les restes alkyle, cycloalkyle, aryle, alkylaryle et arylalkyle,
- $R_4$, $R_8$, $R_8$ et $R_7$ sont choisis parmi l'atome d'hydrogène et les restes alkyle, et

- a et b sont choisis parmi les nombres entiers de 1 à 3.

**2.** Procédé de fabrication des composés représentés par la formule (Ia) ou (Ib) ou (Ic), qui est telle que définie à la revendication 1 et dans laquelle E représente un reste

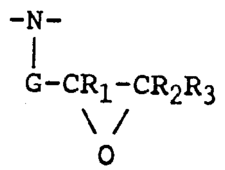 ,

caractérisé par le fait que :

- dans une première étape, on fait réagir un composé de formule :

A-NH-D-$NH_2$ (IIa) ou

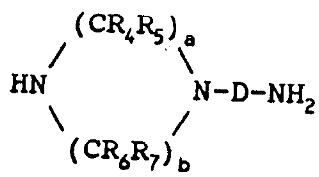 (IIb)

ou

$H_2N$-D-NH-D-$NH_2$ (IIc)

avec un composé carbonylé de formule :

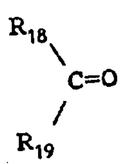

dans laquelle $R_{18}$ et $R_{19}$ sont tels que définis ci-dessus pour $R_1$, avec la condition qu'ils ne sont pas tous deux l'atome d'hydrogène, pour obtenir le composé de formule:

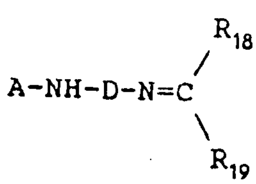 (IVa)

ou

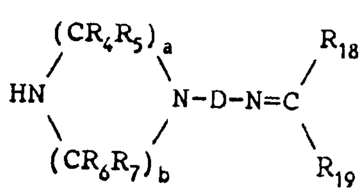 (IVb)

ou

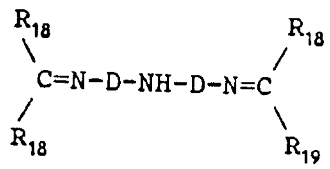

(IVc),

- dans une seconde étape, on fait réagir le composé (IVa) ou (IVb) ou (IVc) avec le disulfure de carbone en présence d'au moins un agent oxydant, pour obtenir le composé :

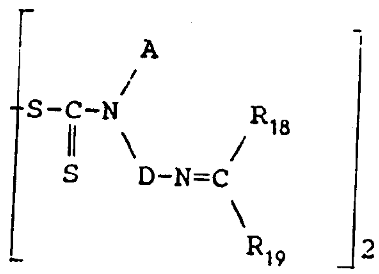

(Va)

ou

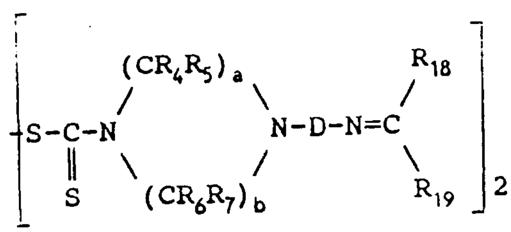

(Vb)

ou

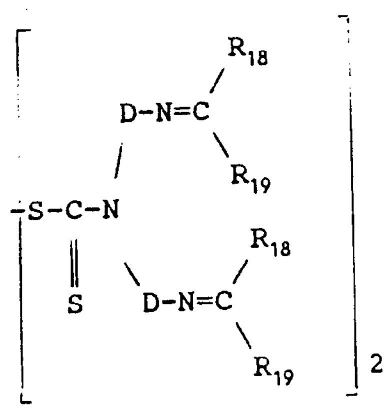

(Vc),

- dans une troisième étape, on hydrolyse ou on réduit le composé (Va) ou (Vb) ou (Vc) pour obtenir le composé de formule :

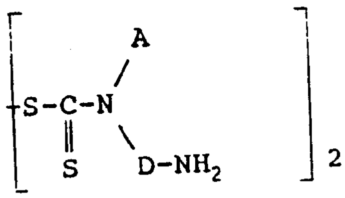

(VIa)

ou

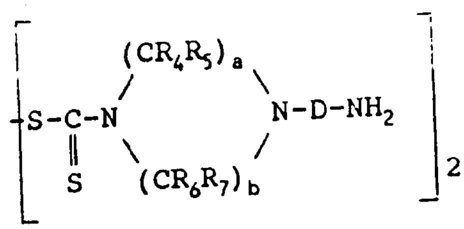

(VIb)

ou

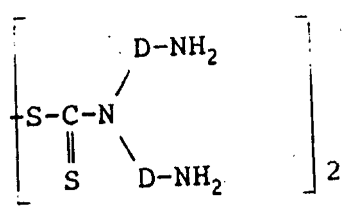

(VIc),

puis

- dans une quatrième étape, on fait réagir le composé (VIa) ou (VIb) ou (VIc) en milieu basique, avec un époxyde de formule :

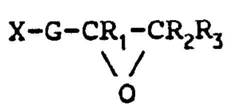

(VII)

dans laquelle X représente un atome d'halogène, le rapport molaire du composé (VII) au composé (VIa) ou (VIb) ou (VIc) étant au moins égal à 2.

3. Procédé de fabrication de composés représentés par la formule (Ia) ou (Ib) ou (Ic), qui est telle que définie à la revendication 1 et dans laquelle E représente -J-K-, K représentant une simple liaison, caractérisé par le fait que :
   - dans une première étape, on fait réagir un composé de formule :

A-NH-D-JH (VIIIa) ou

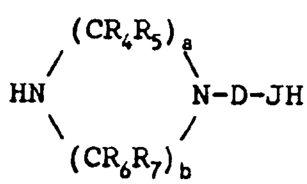

(VIIIb)

ou

HJ-D-NH-D-JH (VIIIc)

ou bien l'un de ses sels d'ammonium quaternaire avec le disulfure de carbone, en présence d'au moins un agent oxydant, pour obtenir le composé :

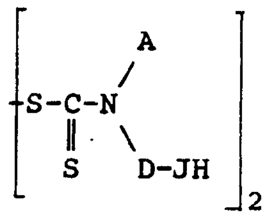

(IXa)

ou

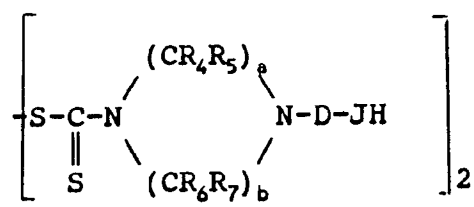

(IXb)

ou

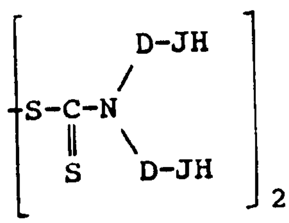

(IXc)

- puis dans une seconde étape, on fait réagir le composé (IXa) ou (IXb) ou (IXc) en milieu basique avec un excès molaire de l'époxyde de formule (VII) :

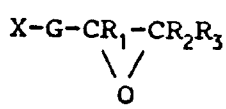

(VII)

dans laquelle X représente un atome d'halogène.

4. Procédé de fabrication de composés représentés par la formule (Ia) ou (Ic), qui est telle que définie à la revendication 1 et dans laquelle E représente -J-K, K représentant un reste

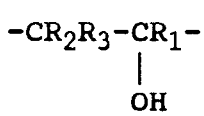

ou

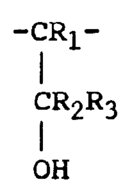

,

caractérisé par le fait que :

- dans une première étape, on fait réagir un composé de formule (VIIIa) ou (VIIIc) avec un haloacide HX, pour obtenir le sel de formule :

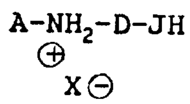 (Xa) ;

ou

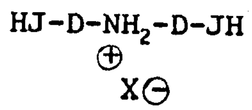 (Xc)

- dans une seconde étape, on fait réagir le sel (Xa) ou (Xc) en milieu basique avec un diépoxyde de formule :

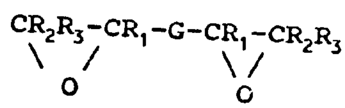 (XI)

pour obtenir un mélange statistique des composés de formules :

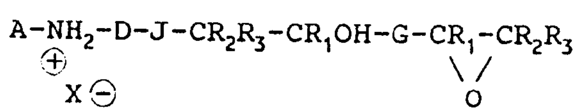 (XIIa)

et

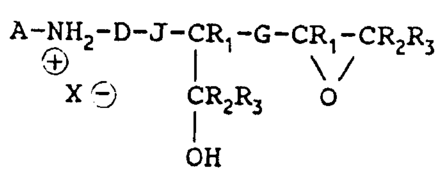 (XIIIa) ;

ou bien

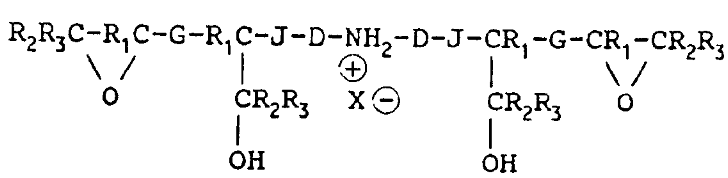 (XIIIc) ;

et

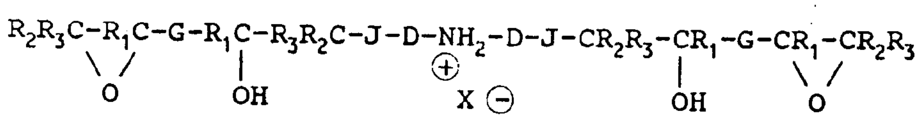 (XIIc) ;

- puis dans une troisième étape, on fait réagir les composés (XIIa) et (XIIIa) ou bien (XIIc) et (XIIIc) avec le disulfure de carbone, en présence d'au moins un agent oxydant.

**5.** Procédé de fabrication de composés représentés par la formule (Ia) ou (Ic) qui est telle que définie à la revendication 1 et dans laquelle J représente l'atome d'oxygène et K représente une simple liaison ou un reste

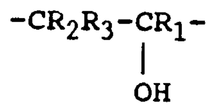 ou 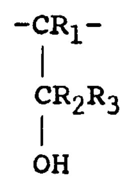 ,

caractérisé en ce qu'on fait réagir un disulfure de thiurame de formule :

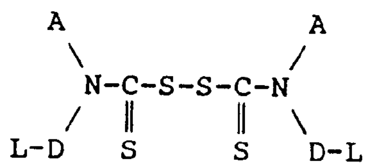 (XVa),

ou

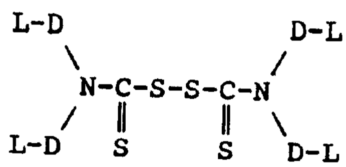 (XVc),

L désignant un groupe hydroxyle ou bien $NHR_{20}$ dans lequel $R_{20}$ est tel que défini pour $R_4$, avec un époxyde de formule :

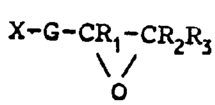 (VII)

dans laquelle X représente un atome d'halogène, ou bien avec un diépoxyde de formule :

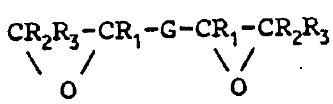 (XI)

6. Utilisation d'un disulfure de thiurame fonctionnel tel que défini à la revendication 1, comme agent ayant la triple fonction d'initiateur, d'agent de transfert de chaîne et d'agent de terminaison, dans la polymérisation radiculaire de monomères vinyliques.

7. Oligomères et polymères de monomère vinylique Mv, ayant pour formule :

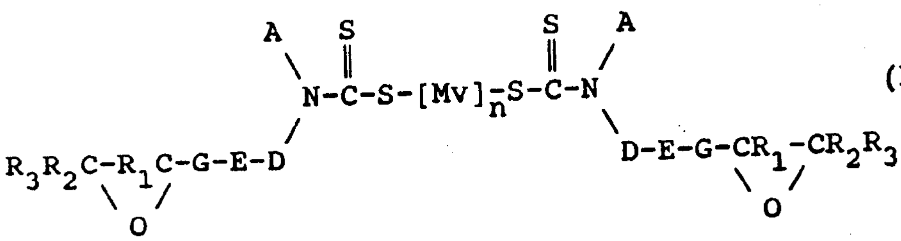

(XIVa),

ou

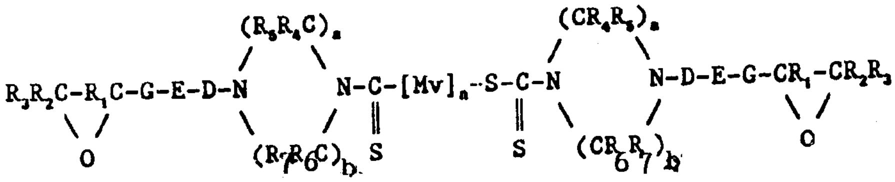

(XIVb),

ou

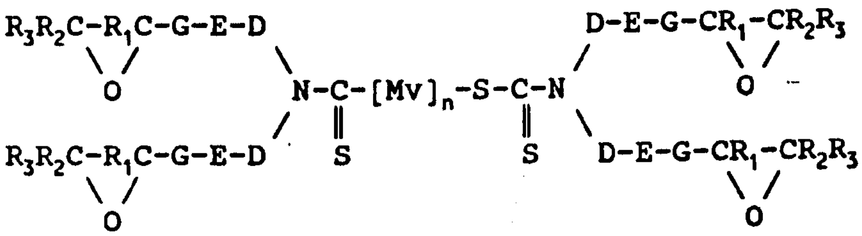

(XIVc)

dans laquelle :

- D, G, E, $R_1$, $R_2$, $R_3$, $R_4$, $R_5$, $R_6$, $R_7$, a et b sont tels que définis à la revendication 1 ; et
- n est un nombre entier tel que la masse moléculaire moyenne en nombre de la séquence dérivée de Mv soit comprise entre $10^2$ et $10^6$.

8. Oligomères et polymères selon la revendication 7, caractérisés en ce que Mv est choisi parmi les acrylates et méthacrylates d'alkyle, dont le groupe alkyle comprend le cas échéant au moins un hétéroatome sous forme d'un atome d'halogène, d'un groupe carboxyle ou d'un groupe amino, et d'aryle,les hydrocarbures vinylaromatiques, les acrylates et méthacrylates époxydés, les acrylamides et méthacrylamides, les acrylates et méthacrylates de dialkylaminoalkyle et leurs sels quaternaires, les nitriles insaturés, les maléimides N-substituées, les diènes, les halogénures de vinyle et de vinylidène, les esters vinyliques, les méthacrylates de trialkylsilyle et de trialcoxysilyle, le 9-vinylcarbazole, la vinyl-2-pyridine, la vinyl-4-pyridine, la 1-vinyl-2-pyrrolidinone, le vinyltriméthoxysilane, le vinyltriméthylsilane et le 1-vinylimidazole.

9. Utilisation selon la revendication 6, caractérisée par le fait que la quantité d'iniferter introduite est comprise entre 5 x $10^{-5}$ mole/l et 0,1 mole/l par rapport au monomère vinylique.

10. Oligomères et polymères selon la revendication 7, caractérisés en ce que n est un nombre entier tel que la masse moléculaire moyenne en nombre de la séquence dérivée de Mv soit comprise entre $10^2$ et $10^6$.

**Revendications pour l'Etat contractant suivant : ES**

1. Procédé de fabrication de disulfures de thiurame fonctionnels, représentés par les formules (Ia) ou (Ib) ou (Ic) :

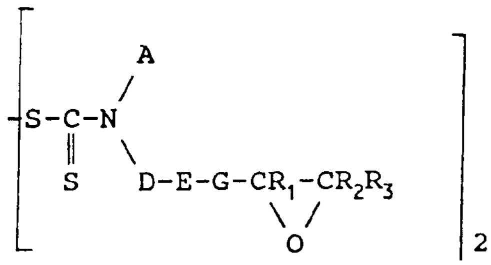

(Ia)

ou

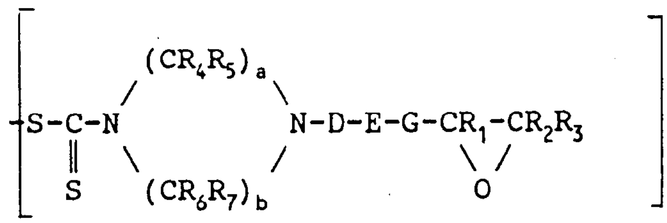

(Ib)

ou encore

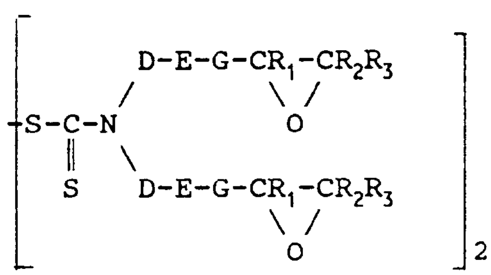

(Ic)

dans lesquelles :

- A est choisi parmi les groupes alkyle, cycloalkyle, aryle, arylalkyle, alkylaryle, les séquences polyoxyalkylène représentées par les formules suivantes :

$$R_{10}\text{-O-(-}CH_2\text{-}CH_2\text{-O-)}_c\text{-}CH_2\text{-}CH_2\text{-}$$

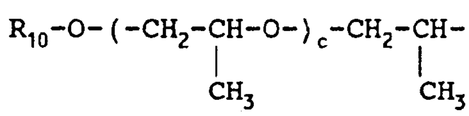

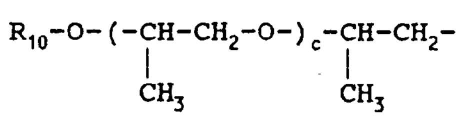

$$R_{10}\text{-O-[-}(CH_2)_4\text{-O-]}_c\text{-}(CH_2)_4\text{-}$$

où :

- $R_{10}$ représente un reste alkyle, cycloalkyle ou aryle ; et

- c vaut de 1 à 1000 ;

et les séquences polyester représentées par les formules suivantes :

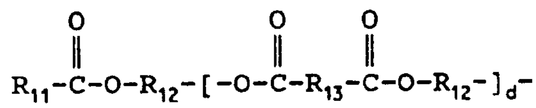

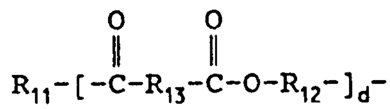 ;

et

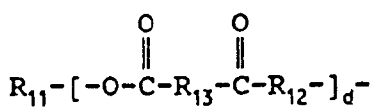

où :

- $R_{11}$ est tel que défini ci-dessus pour $R_{10}$ ;
- $R_{12}$ et $R_{13}$ sont choisis parmi les groupes alkylène, cycloalkylène, arylène, alcénylène, alcénylarylène et peuvent comprendre au moins un atome de nature différente au sein de la chaîne et/ou du cycle et/ou au moins un reste

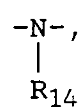

  $R_{14}$ étant choisi parmi les groupes alkyle, cycloalkyle et aryle, et pouvant comporter des substituants ; et
- d vaut de 1 à 300,

- D et G sont choisis parmi les groupes alkylène, cycloalkylène, arylène, alkylarylène et arylalkylène et peuvent comporter au moins un atome de nature différente au sein de la chaîne et/ou du cycle,
- E représente un reste

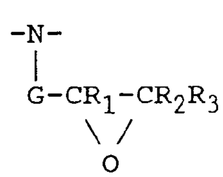

- $R_1$, $R_2$, $R_3$ sont choisis parmi l'atome d'hydrogène et les restes alkyle, cycloalkyle, aryle, alkylaryle et arylalkyle,
- $R_4$, $R_5$, $R_5$ et $R_7$ sont choisis parmi l'atome d'hydrogène et les restes alkyle, et
- a et b sont choisis parmi les nombres entiers de 1 à 3,

caractérisé par le fait que :

- dans une première étape, on fait réagir un composé de formule :

$A\text{-}NH\text{-}D\text{-}NH_2$ (IIa) ou

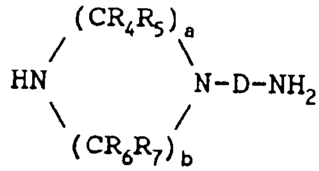

(IIb)

ou

$H_2N\text{-}D\text{-}NH\text{-}D\text{-}NH_2$ (IIc)

avec un composé carbonylé de formule :

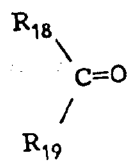

dans laquelle $R_{18}$ et $R_{19}$ sont tels que définis ci-dessus pour $R_1$, avec la condition qu'ils ne sont pas tous deux l'atome d'hydrogène, pour obtenir le composé de formule:

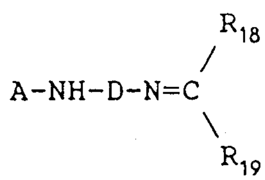

(IVa)

ou

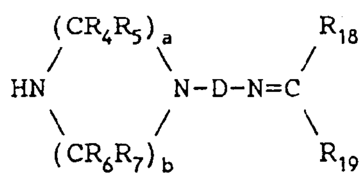

(IVb)

ou

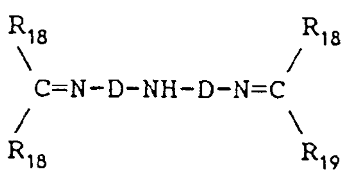

(IVc),

- dans une seconde étape, on fait réagir le composé (IVa) ou (IVb) ou (IVc) avec le disulfure de carbone en présence d'au moins un agent oxydant, pour obtenir le composé :

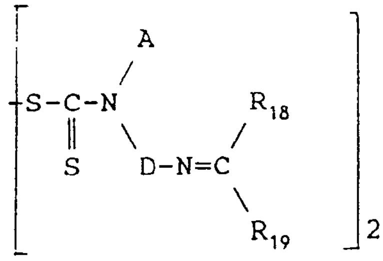

(Va)

ou

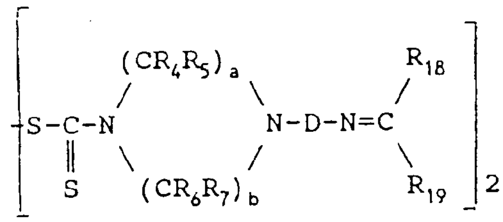

(Vb)

ou

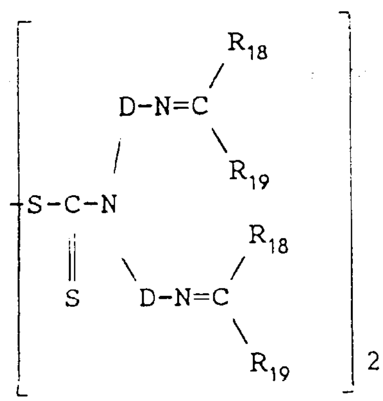

(Vc) ,

- dans une troisième étape, on hydrolyse ou on réduit le composé (Va) ou (Vb) ou (Vc) pour obtenir le composé de formule :

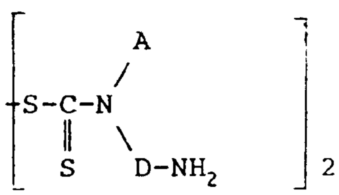

(VIa)

ou

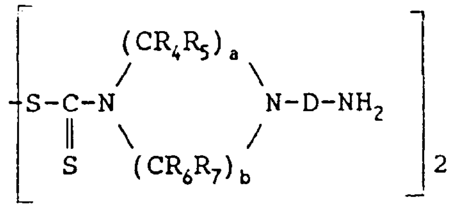

(VIb)

ou

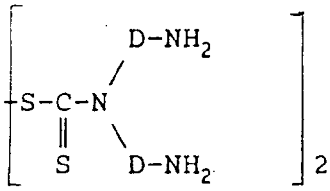

(VIc),

puis

- dans une quatrième étape, on fait réagir le composé (VIa) ou (VIb) ou (VIc) en milieu basique, avec un époxyde de formule :

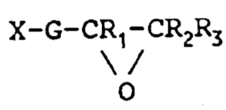

(VII)

dans laquelle X représente un atome d'halogène, le rapport molaire du composé (VII) au composé (VIa) ou (VIb) ou (VIc) étant au moins égal à 2.

**2.** Procédé de fabrication de disulfures de thiurame représentés par les formules (Ia) ou (Ib) ou (Ic), dans lesquelles :

- A, D, G, $R_1$ à $R_7$, a et b sont tels que définis à la revendication 1 ; et
- E représente un reste -J-K-, J étant choisi parmi les groupements $CR_8R_9O$, $R_8$ et $R_9$ étant choisis parmi l'atome d'hydrogène, et les restes alkyle, cycloalkyle, aryle, alkylaryle et arylalkyle,

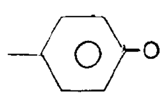 et 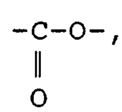,

et

K représentant une simple liaison,

caractérisé par le fait que :

- dans une première étape, on fait réagir un composé de formule :

A-NH-D-JH (VIIIa) ou

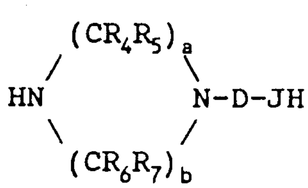

(VIIIb)

ou

HJ-D-NH-D-JH (VIIIc)

ou bien l'un de ses sels d'ammonium quaternaire avec le disulfure de carbone, en présence d'au moins un agent oxydant, pour obtenir le composé :

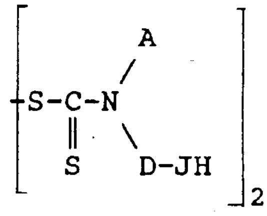

(IXa)

ou

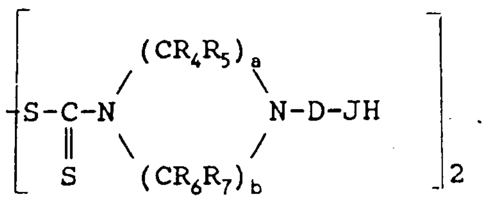

(IXb)

ou

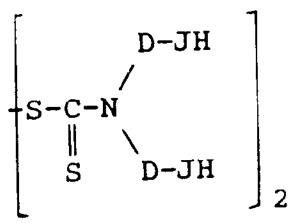

(IXc)

- puis dans une seconde étape, on fait réagir le composé (IXa) ou (IXb) ou (IXc) en milieu basique avec un excès molaire de l'époxyde de formule (VII) :

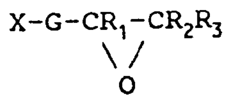

(VII)

dans laquelle X représente un atome d'halogène.

3. Procédé de fabrication de disulfures de thiurame représentés par les formules (Ia) ou (Ic) dans lesquelles :
   - A, D, G, $R_1$ à $R_7$, a et b sont tels que définis à la revendication 1 ; et
   - E représente -J-K, J représentant $CR_8R_9O$, $R_8$ et $R_9$ étant tels que définis à la revendication 2,

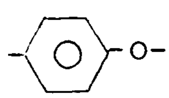 , 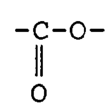

et K représentant un reste :

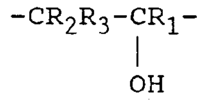 ou 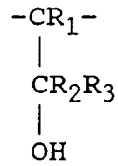 ,

caractérisé par le fait que :

- dans une première étape, on fait réagir un composé de formule (VIIIa) ou (VIIIc) avec un haloacide HX, pour obtenir le sel de formule :

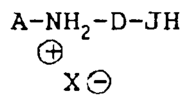 (Xa) ;

ou

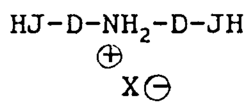 (Xc)

- dans une seconde étape, on fait réagir le sel (Xa) ou (Xc) en milieu basique avec un diépoxyde de formule :

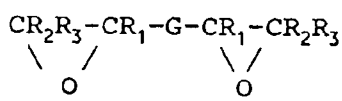 (XI)

pour obtenir un mélange statistique des composés de formules :

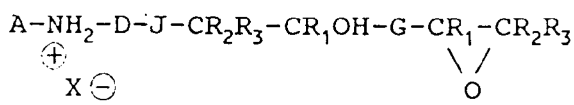 (XIIa)

et

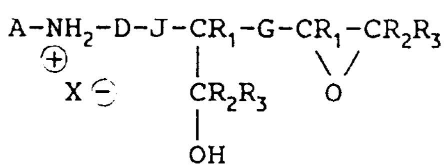 (XIIIa) ;

ou bien

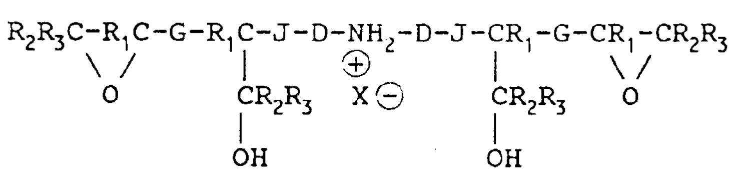 (XIIIc) ;

et

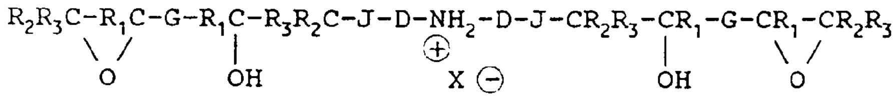 (XIIc) ;

- puis dans une troisième étape, on fait réagir les composés (XIIa) et (XIIIa) ou bien (XIIc) et (XIIIc) avec le disulfure de carbone, en présence d'au moins un agent oxydant.

**4.** Procédé de fabrication de disulfures de thiurame représentés par les formules (Ia) ou (Ic) dans lesquelles :
- A, D, G, $R_1$ à $R_7$, a et b sont tels que définis à la revendication 1 ; et
- E représente -J-K, J représentant l'atome d'oxygène et K représentant une simple liaison ou un reste

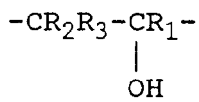 ou 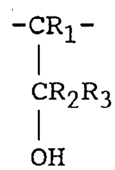 ,

caractérisé par le fait qu'on fait réagir un disulfure de thiurame de formule :

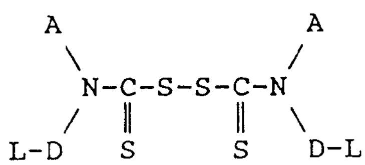 (XVa) ,

ou

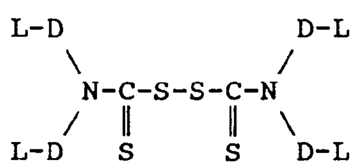 (XVc) ,

L désignant un groupe hydroxyle ou bien $NHR_{20}$ dans lequel $R_{20}$ est tel que défini pour $R_4$, avec un époxyde de formule :

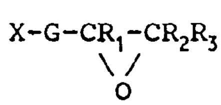 (VII)

dans laquelle X représente un atome d'halogène, ou bien avec un diépoxyde de formule :

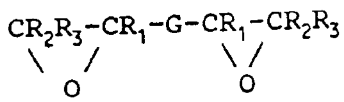

(XI)

5. Procédé de polymérisation radicalaire d'au moins un monomère vinylique Mv pour obtenir des oligomères et polymères de monomère vinylique Mv, ayant pour formule :

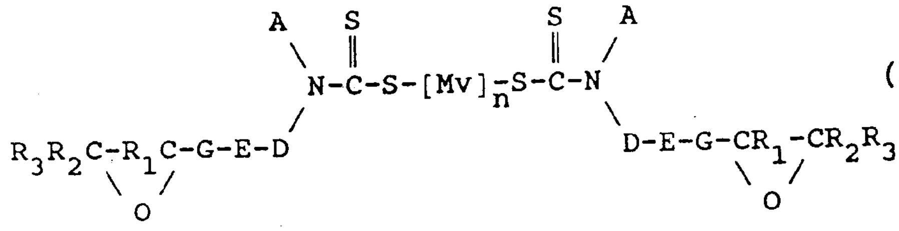

(XIVa),

ou

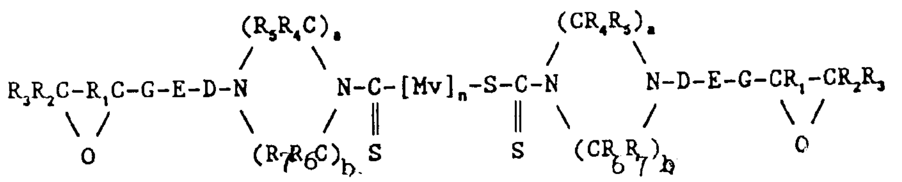

(XIVb),

ou

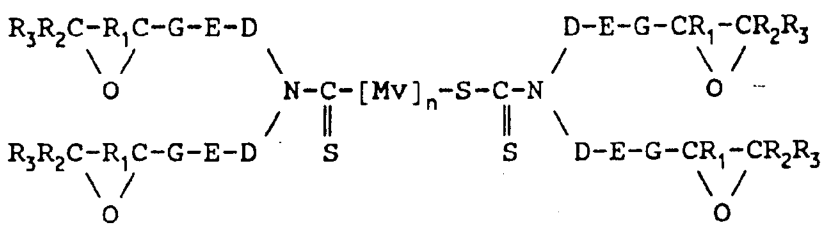

(XIVc)

dans laquelle :

- A, D, G, $R_1$, $R_2$, $R_3$, $R_4$, $R_5$, $R_6$, $R_7$, a et b sont tels que définis à la revendication 1 ;
- E est tel que défini aux revendications 1 et/ou 2 et/ou 3 et/ou 4 ; et
- n est un nombre entier tel que la masse moléculaire moyenne en nombre de la séquence dérivée de Mv soit comprise entre $10^2$ et $10^6$,

caractérisé par le fait qu'on utilise au moins un disulfure de thiurame fonctionnel préparé par un procédé tel que défini aux revendications 1 et/ou 2 et/ou 3 et/ou 4, comme agent ayant la triple fonction d'initiateur, d'agent de transfert de chaîne et d'agent de terminaison, dans la polymérisation radicalaire de monomères vinyliques.

6. Procédé selon la revendication 5, caractérisé par le fait que la quantité d'iniferter introduite est comprise entre 5 x $10^{-5}$ mole/l et 0,1 mole/l par rapport au monomère vinylique.

7. Procédé selon la revendication 5, caractérisé en ce qu'on choisit Mv parmi les acrylates et méthacrylates d'alkyle, dont le groupe alkyle comprend le cas échéant au moins un hétéroatome sous forme d'un atome d'halogène, d'un groupe carboxyle ou d'un groupe amino, et d'aryle, les hydrocarbures vinylaromatiques,

les acrylates et méthacrylates époxydés, les acrylamides et méthacrylamides, les acrylates et méthacrylates de dialkylaminoalkyle et leurs sels quaternaires, les nitriles insaturés, les maléimides N-substituées, les diènes, les halogénures de vinyle et de vinylidène, les esters vinyliques, les méthacrylates de trialkylsilyle et de trialcoxysilyle, le 9-vinylcarbazole, la vinyl-2-pyridine, la vinyl-4-pyridine, la 1-vinyl-2-pyrrolidinone, le vinyltriméthoxysilane, le vinyltriméthylsilane et le 1-vinylimidazole.

8. Procédé selon l'une des revendications 5 à 7, caractérisé par le fait qu'il conduit à un oligomère ou polymère dans lequel n est un nombre entier tel que la masse moléculaire moyenne en nombre de la séquence dérivée de Mv soit comprise entre $10^2$ et $10^6$.

## Claims

### Claims for the following Contracting States : AT, BE, CH, DE, DK, FR, GB, GR, IT, LI, LU, NL, SE

1. Functional thiuram disulphides of formula :

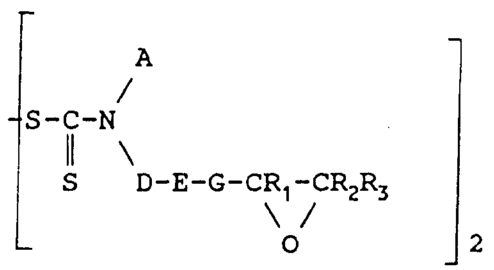

(Ia)

or

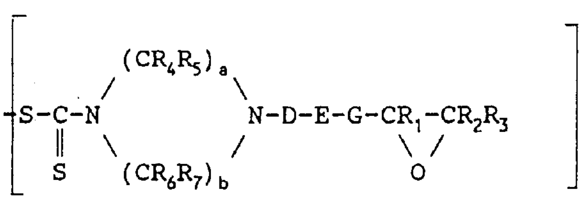

(Ib)

or

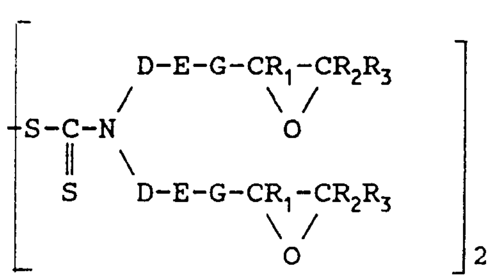

(Ic)

in which :

- A is chosen from alkyl, cycloalkyl, aryl, arylalkyl and alkylaryl groups, polyoxyalkylene sequences represented by the following formulae :

$$R_{10}\text{-O-(-CH}_2\text{-CH}_2\text{-O-)}_c\text{-CH}_2\text{-CH}_2\text{-}$$

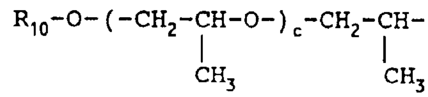

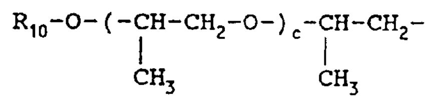

$$R_{10}\text{-O-[-}CH_2)_4\text{-O-]}_c\text{-}(CH_2)_4\text{-}$$

in which :

- $R_{10}$ represents an alkyl, cycloalkyl or aryl radical ; and
- c ranges from 1 to 1000 ;

and polyester sequences represented by the following formulae :

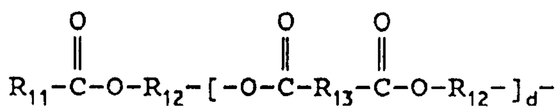

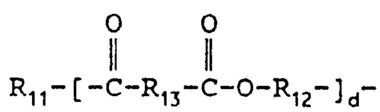 ;

and

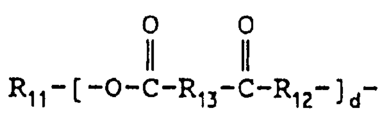

in which :

- $R_{11}$ is as defined above for $R_{10}$
- $R_{12}$ and $R_{13}$ are chosen from alkylene, cycloalkylene, arylene, alkenylene, alkenylarylene groups and may contain at least one atom having a different nature within the chain and/or the ring and/or at least one

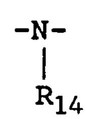

radical, $R_{14}$ being chosen from alkyl, cycloalkyl and aryl groups, and it being possible for $R_{14}$ to have substituents ; and
- d ranges from 1 to 300,

- D and G are chosen from alkylene, cycloalkylene, arylene, alkylarylene and arylalkylene groups and may contain at least one atom having a different nature within the chain and/or the ring,
- E represents :
  . a

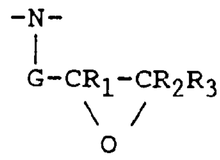

radical, or

. a -J-K- radical, J being chosen from the $CR_8R_9O$,

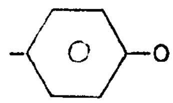 and 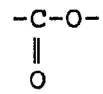

groups, and K representing a single bond, or, in the case of the compounds (Ia), J may represent an oxygen atom and K may represent a

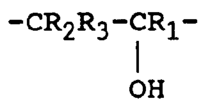 (a), or 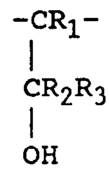 (b)

radical,

- $R_1$, $R_2$, $R_3$, $R_8$ and $R_9$ are chosen from a hydrogen atom and alkyl, cycloalkyl, aryl, alkylaryl and arylalkyl radicals,
- $R_4$, $R_5$, $R_5$ and $R_7$ are chosen from a hydrogen atom and alkyl radicals, and
- a and b are chosen from integers from 1 to 3.

**2.** Process for the production of the compounds represented by the formula (Ia) or (Ib) or (Ic), which is as defined in Claim 1 and in which E represents a

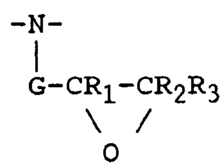

radical, characterised in that :

- in a first step, a compound of formula :

$$A\text{-}NH\text{-}D\text{-}NH_2 \qquad \text{(IIa) or}$$

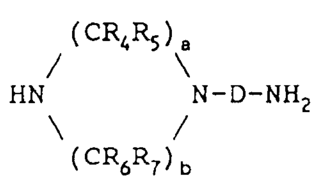 (IIb)

or

$$H_2N\text{-}D\text{-}NH\text{-}D\text{-}NH_2 \qquad \text{(IIc)}$$

is reacted with a carbonyl compound of formula :

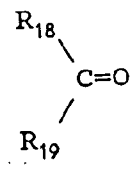

in which $R_{18}$ and $R_{19}$ are as defined above for $R_1$, on condition that they are not both a hydrogen atom, in order to obtain a compound of formula :

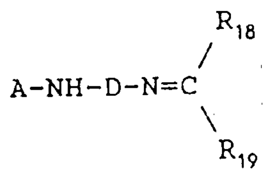

(IVa)

or

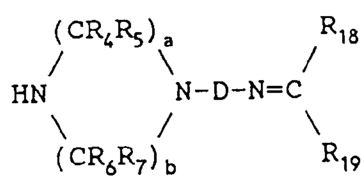

(IVb)

or

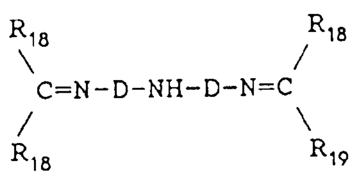

(IVc) ,

- in a second step, the compound (IVa) or (IVb) or (IVc) is reacted with carbon disulphide in the presence of at least one oxidising agent, in order to obtain the compound :

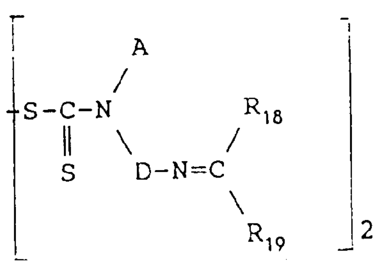

(Va)

or

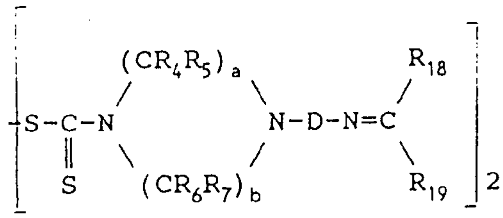

(Vb)

or

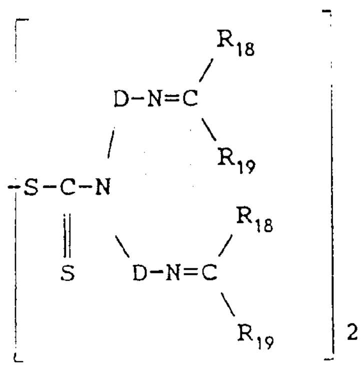

(Vc),

- in a third step, the compound (Va) or (Vb) or (Vc) is hydrolysed or reduced in order to obtain the compound of formula :

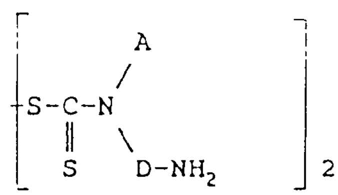

(VIa)

or

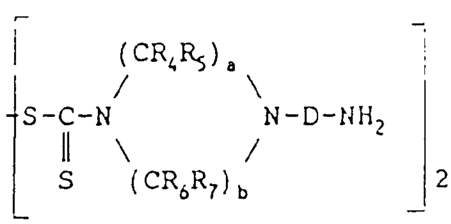

(VIb)

or

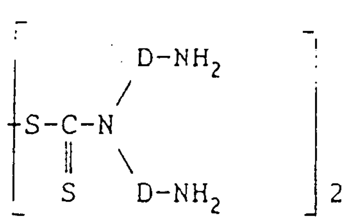

(VIc),

and then

- in a fourth step, the compound (VIa) or (VIb) or (VIc) is reacted in a basic medium with an epoxide of formula:

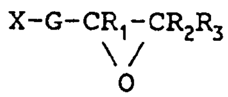

(VII)

in which X represents a halogen atom, the molar ratio of the compound (VII) to the compound (VIa) or (VIb) or (VIc) being at least 2.

**3.** Process for the production of compounds represented by the formula (Ia) or (Ib) or (Ic), which is as defined in Claim 1 and in which E represents -J-K-, K representing a single bond, characterised in that :

- in a first step, a compound of formula :

A-NH-D-JH (VIIIa) or

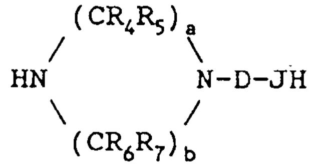

(VIIIb)

or

HJ-D-NH-D-JH (VIIIc)

or one of its quaternary ammonium salts is reacted with carbon disulphide, in the presence of at least one oxidising agent, in order to obtain the compound :

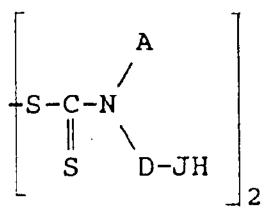

(IXa)

or

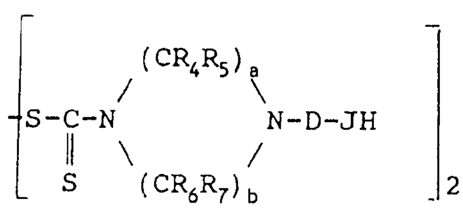

(IXb)

or

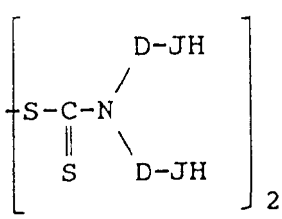

(IXc)

- and then, in a second step, the compound (IXa) or (IXb) or (IXc) is reacted in a basic medium with a molar excess of the epoxide of formula (VII)

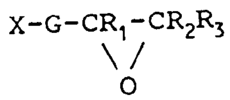

(VII)

in which X represents a halogen atom.

**4.** Process for the production of compounds represented by the formula (Ia) or (Ic), which is as defined in Claim 1 and in which E represents -J-K, K representing a

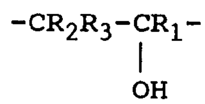

or

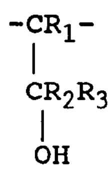

radical,
characterised in that :

- in a first step, a compound of formula (VIIIa) or (VIIIc) is reacted with a haloacid HX in order to obtain the salt of formula :

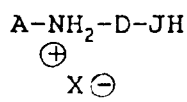

(Xa) ;

or

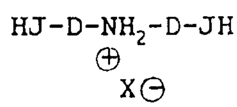

(Xc)

- in a second step, the salt (Xa) or (Xc) is reacted in a basic medium with a diepoxide of formula :

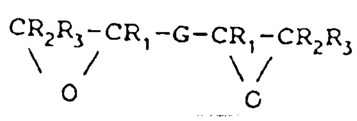

(XI)

in order to obtain a random mixture of the compounds of formulae :

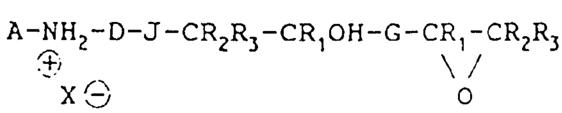

(XIIa)

and

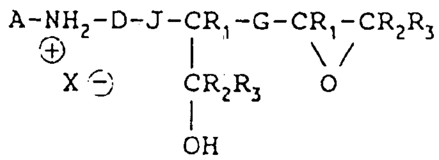 (XIIIa) ;

or

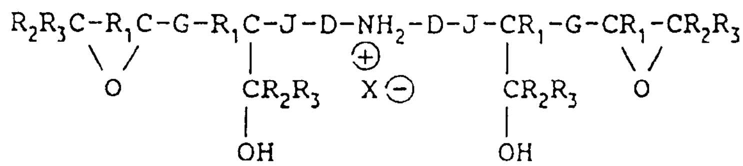 (XIIIc) ;

and

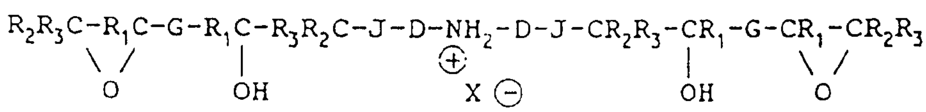 (XIIc) ;

- and then, in a third step, the compounds (XIIa) and (XIIIa) or (XIIc) and (XIIIc) are reacted with carbon disulphide in the presence of at least one oxidising agent.

5. Process for the production of compounds represented by the formula (Ia) or (Ic) which is as defined in Claim 1 and in which J represents an oxygen atom and K represents a single bond or a

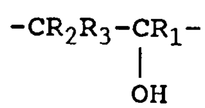 or 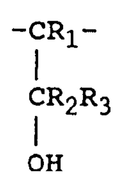

radical, characterised in that a thiuram disulphide of formula :

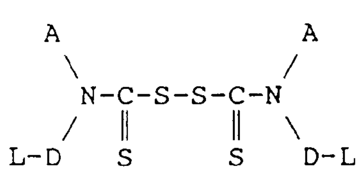 (XVa),

or

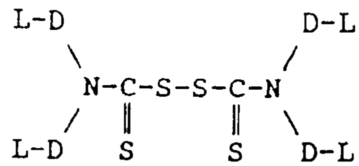

(XVc) ,

L denoting a hydroxyl group or $NHR_{20}$, in which $R_{20}$ is as defined for $R_4$, is reacted with an epoxide of formula :

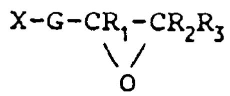

(VII)

in which X represents a halogen atom, or with a diepoxide of formula :

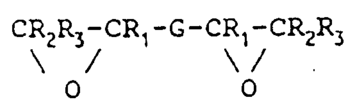

(XI)

6. Use of a functional thiuram disulphide as defined in Claim 1 as agent having the triple function of initiator, chain transfer agent and terminating agent in the free radical polymerisation of vinyl monomers.

7. Oligomers and polymers of a vinyl monomer Mv, having the formula :

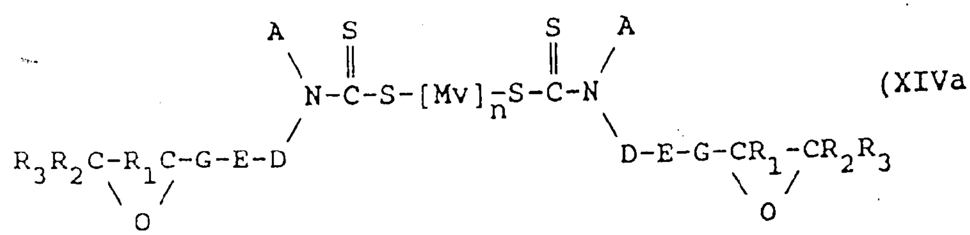

(XIVa) ,

or

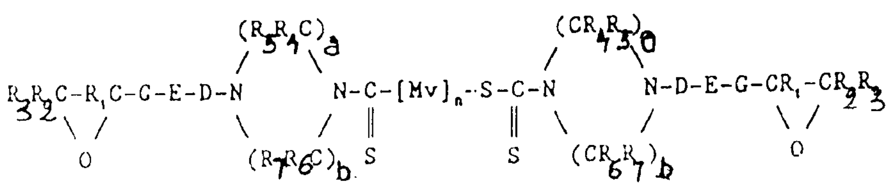

(XIVb) ,

or

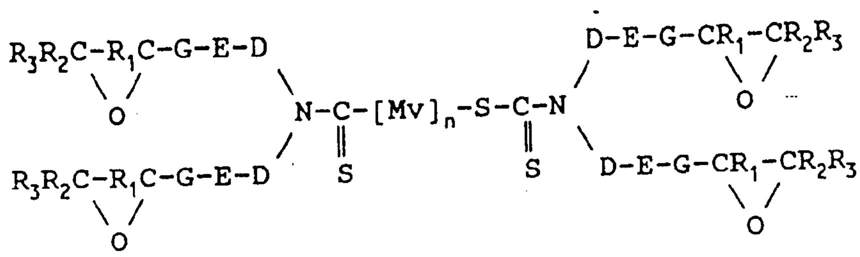

(XIVc)

in which :

- D, G, E, $R_1$, $R_2$, $R_3$, $R_4$, $R_5$, $R_6$, $R_7$, a and b are as defined in Claim 1 ; and
- n is an integer such that the number-average molecular mass of the sequence derived from Mv is between $10^2$ and $10^6$.

**8.** Oligomers and polymers according to Claim 7, characterised in that Mv is chosen from alkyl acrylates and methacrylates, the alkyl group of which contains, if appropriate, at least one hetero-atom as a halogen atom, a carboxyl group or an amino group, and aryl acrylates and methacrylates, vinyl-aromatic hydrocarbons, epoxy acrylates and methacrylates, acrylamides and methacrylamides, dialkylaminoalkyl acrylates and methacrylates and their quaternary salts, unsaturated nitriles, N-substituted maleimides, dienes, vinyl halides and vinylidene halides, vinyl esters, trialkylsilyl methacrylates and trialkoxysilyl methacrylates, 9-vinyl-carbazole, vinyl-2-pyridine, vinyl-4-pyridine, 1-vinyl-2-pyrrolidinone, vinyltrimethoxysilane, vinyltrimethylsilane and 1-vinylimidazole.

**9.** Use according to Claim 6, characterised in that the amount of iniferter introduced is between $5 \times 10^{-5}$ mol/l and 0.1 mol/l with respect to the vinyl monomer.

**10.** Oligomers and polymers according to Claim 7, characterised in that n is an integer such that the number-average molecular mass of the sequence derived from Mv is between $10^2$ and $10^6$.

**Claims for the following Contracting State : ES**

**1.** Process for the production of functional thiuram disulphides, represented by the formulae (Ia) or (Ib) or (Ic) :

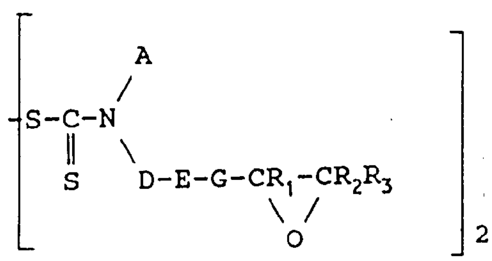

(Ia)

or

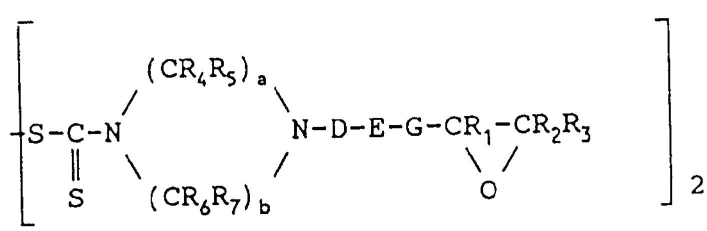

(Ib)

or

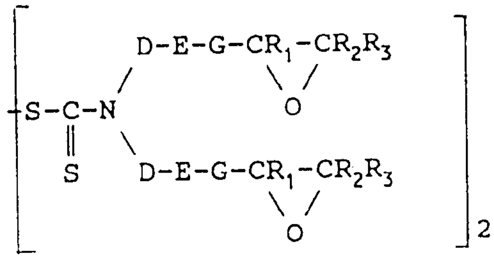

(Ic)

in which :

- A is chosen from alkyl, cycloalkyl, aryl, arylalkyl and alkylaryl groups, polyoxyalkylene sequences represented by the following formulae :

$$R_{10}\text{-O-(-}CH_2\text{-}CH_2\text{-O-)}_c\text{-}CH_2\text{-}CH_2\text{-}$$

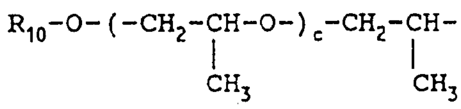

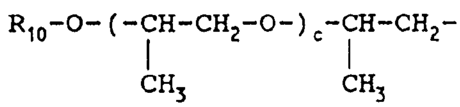

$$R_{10}\text{-O-[-}(CH_2)_4\text{-O-]}_c\text{-}(CH_2)_4\text{-}$$

in which :

- $R_{10}$ represents an alkyl, cycloalkyl or aryl radical ; and
- c ranges from 1 to 1000 ;

and polyester sequences represented by the following formulae :

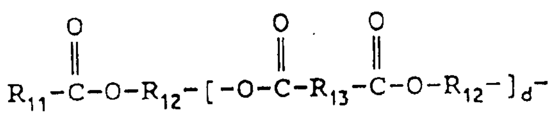

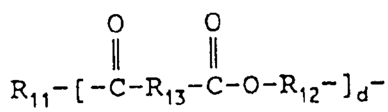

;

and

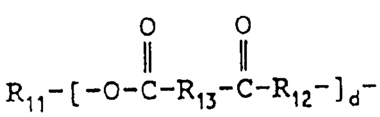

in which :

- $R_{11}$ is as defined above for $R_{10}$ ;
- $R_{12}$ and $R_{13}$ are chosen from alkylene, cycloalkylene, arylene, alkenylene, alkenylarylene groups

and may contain at least one atom having a different nature within the chain and/or the ring and/or at least one

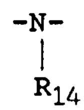

radical, $R_{14}$ being chosen from alkyl, cycloalkyl and aryl groups, and it being possible for $R_{14}$ to have substituents ; and

- d ranges from 1 to 300,

- D and G are chosen from alkylene, cycloalkylene, arylene, alkylarylene and arylalkylene groups and may contain at least one atom having a different nature within the chain and/or the ring,
- E represents a

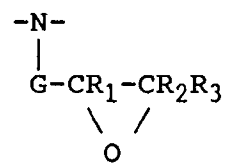

radical, or

- $R_1$, $R_2$, $R_3$ are chosen from a hydrogen atom and alkyl, cycloalkyl, aryl, alkylaryl and arylalkyl radicals,
- $R_4$, $R_5$, $R_5$ and $R_7$ are chosen from a hydrogen atom and alkyl radicals, and
- a and b are chosen from integers from 1 to 3,

characterised in that :

- in a first step, a compound of formula :

$$A\text{-}NH\text{-}D\text{-}NH_2 \qquad \text{(IIa) or}$$

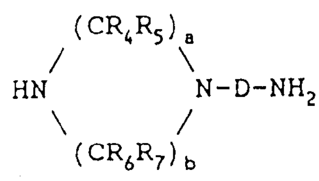 (IIb)

or

$$H_2N\text{-}D\text{-}NH\text{-}D\text{-}NH_2 \qquad \text{(IIc)}$$

is reacted with a carbonvl compound of formula :

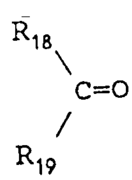

in which $R_{18}$ and $R_{19}$ are as defined above for $R_1$, on condition that they are not both a hydrogen atom, in order to obtain a compound of formula :

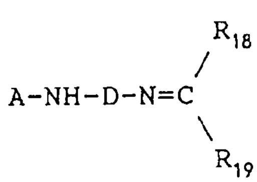 (IVa)

or

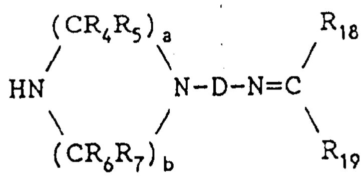

(IVb)

or

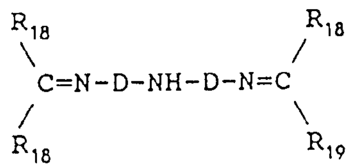

(IVc),

- in a second step, the compound (IVa) or (IVb) or (IVc) is reacted with carbon disulphide in the presence of at least one oxidising agent, in order to obtain the compound :

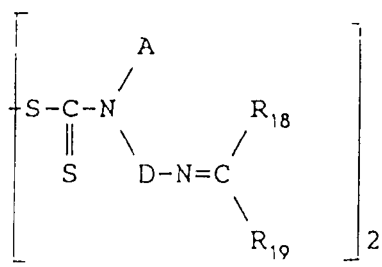

(Va)

or

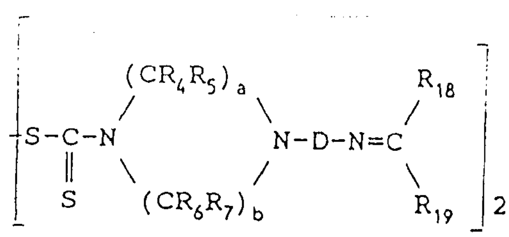

(Vb)

or

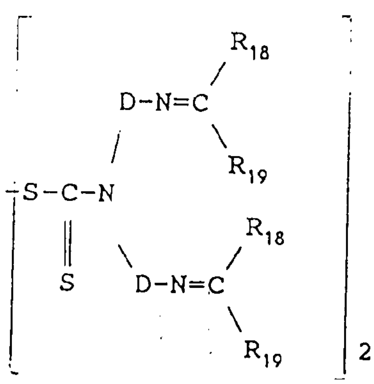

(Vc),

- in a third step, the compound (Va) or (Vb) or (Vc) is hydrolysed or reduced in order to obtain the compound of formula :

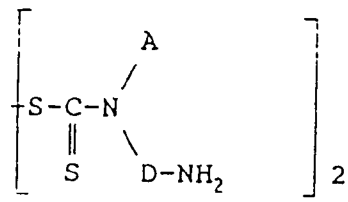

(VIa)

or

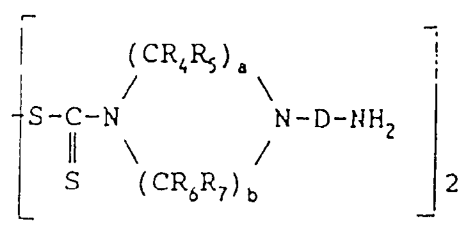

(VIb)

or

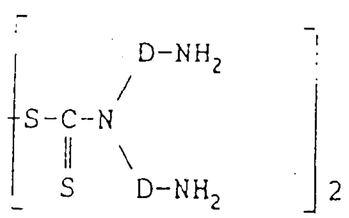

(VIc) ,

and then

- in a fourth step, the compound (VIa) or (VIb) or (VIc) is reacted in a basic medium with an epoxide of formula:

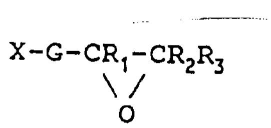

(VII)

in which X represents a halogen atom, the molar ratio of the compound (VII) to the compound (VIa) or (VIb) or (VIc) being at least 2.

**2.** Process for the production of thiuram disulphides represented by the formulae (Ia) or (Ib) or (Ic), in which :

- A, D, G, $R_1$ to $R_7$, a and b are as defined in Claim 1 ; and
- E represents a -J-K- radical, J being chosen from the $CR_8R_9O$, $R_8$ and $R_9$ being chosen from a hydrogen atom and alkyl, cycloalkyl, aryl, alkylaryl and arylalkyl radicals,

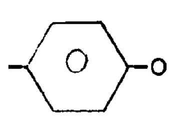

and

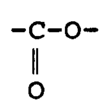

groups, and K representing single bond ,

characterised in that :

- in a first step, a compound of formula :

A-NH-D-JH (VIIIa) or

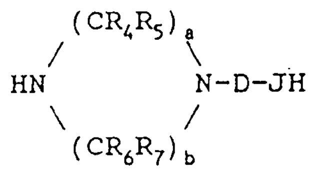

(VIIIb)

or

HJ-D-NH-D-JH (VIIIc)

or one of its quaternary ammonium salts is reacted with carbon disulphide, in the presence of at least one oxidising agent, in order to obtain the compound :

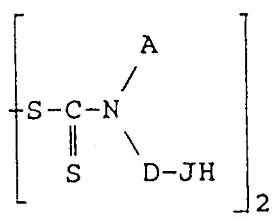

(IXa)

or

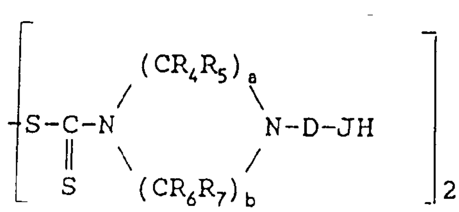

(IXb)

or

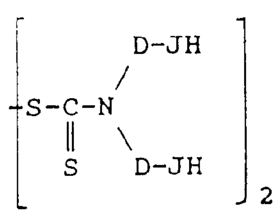

(IXc)

- and then, in a second step, the compound (IXa) or (IXb) or (IXc) is reacted in a basic medium with a molar excess of the epoxide of formula (VII) :

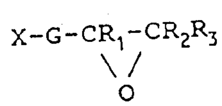

(VII)

in which X represents a halogen atom.

**3.** Process for the production of thiuram disulphides represented by the formulae (Ia) or (Ic), in which :

- A, D, G, $R_1$ to $R_7$, a and b are as defined in Claim 1 ; and
- E represents a -J-K- radical, J representing $CR_8R_9O$, $R_8$ and $R_9$ being as defined in Claim 2,

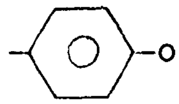

and

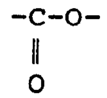 ,

and K representing a

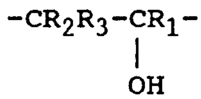 or 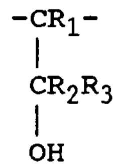

radical,

characterised in that :

- in a first step, a compound of formula (VIIIa) or (VIIIc) is reacted with a haloacid HX in order to obtain the salt of formula :

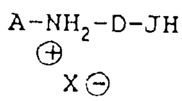

(Xa) ;

or

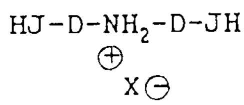

(Xc)

- in a second step, the salt (Xa) or (Xc) is reacted in a basic medium with a diepoxide of formula :

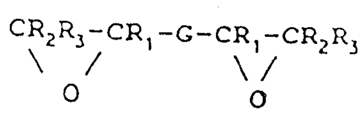

(XI)

in order to obtain a random mixture of the compounds of formulae :

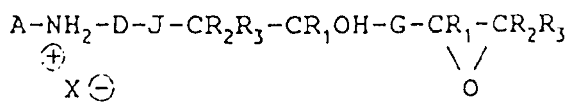 (XIIa)

and

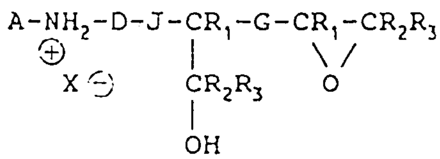 (XIIIa) ;

or

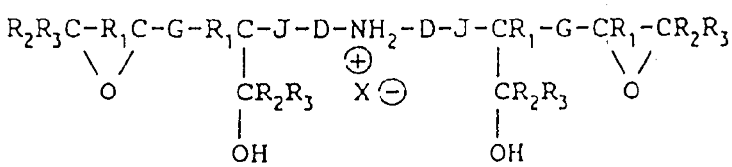 (XIIIc) ;

and

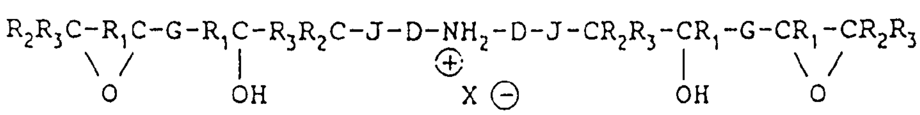 (XIIc) ;

- and then, in a third step, the compounds (XIIa) and (XIIIa) or (XIIc) and (XIIIc) are reacted with carbon disulphide in the presence of at least one oxidising agent.

4. Process for the production of thiuram disulphides represented by the formulae (Ia) or (Ic), in which :
   - A, D, G, $R_1$ to $R_7$, a and b are as defined in Claim 1 ; and
   - E represents a -J-K- radical, J representing an oxygen atom and K representing a single bond or a

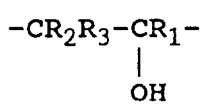 or 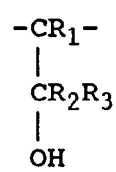

radical,

characterized in that a thiuram disulphide of formula :

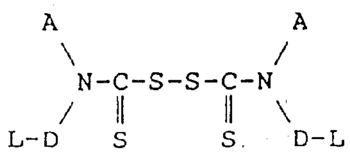

(XVa),

or

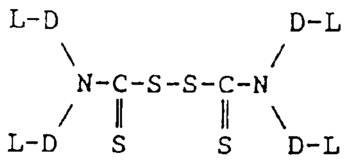

(XVc),

L denoting a hydroxyl group or $NHR_{20}$, in which $R_{20}$ is as defined for $R_4$, is reacted with an epoxide of formula :

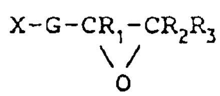

(VII)

in which X represents a halogen atom, or with a diepoxide of formula :

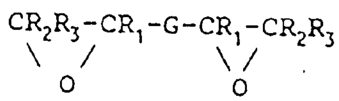

(XI)

5. Process for the radical polymerization of at least one vinyl monomer Mv, in order to obtain oligomers and polymers of a vinyl monomer Mv, having the formula :

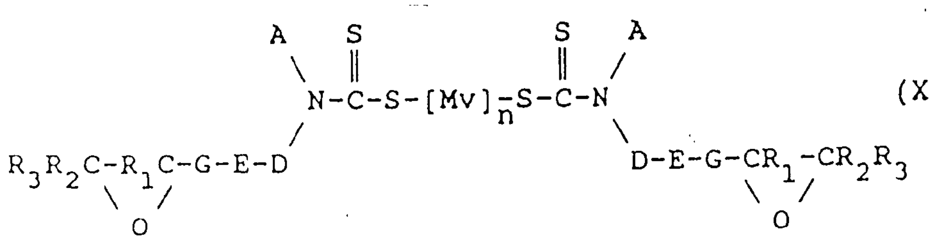

(XIVa) ,

or

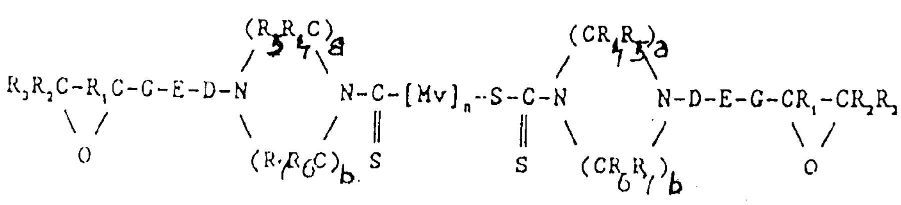

(XIVb) ,

or

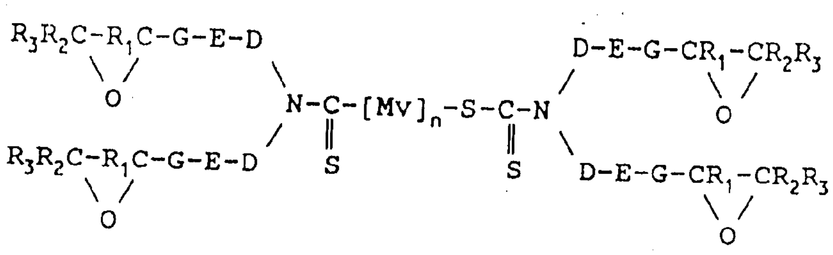

(XIVc)

in which :

- D, G, $R_1$, $R_2$, $R_3$, $R_4$, $R_5$, $R_6$, $R_7$, a and b are as defined in Claim 1 ; and
- E is as defined in claims 1 and/or 2 and/or 3 and/or 4 ;
- n is an integer such that the number-average molecular mass of the sequence derived from Mv is between $10^2$ and $10^6$,

characterized in that at least one functional thiuram disulphide which is obtained by a process as defined in Claim 1 and/or 2 and/or 3 and/or 4 is used, as agent having the triple function of initiator, chain transfer agent and terminating agent, in the free radical polymerisation of vinyl monomers.

6. Process according to claim 5, characterised in that the amount of iniferter introduced is introduced is between 5 x $10^{-5}$ mol/l and 0.1 mol/l with respect to the vinyl monomer.

7. Process according to Claim 5, characterised in that Mv is chosen from alkyl acrylates and methacrylates, the alkyl group of which contains, if appropriate, at least one hetero-atom as a halogen atom, a carboxyl group or an amino group, and aryl acrylates and methacrylates, vinyl-aromatic hydrocarbons, epoxy acrylates and methacrylates, acrylamides and methacrylamides, dialkylaminoalkyl acrylates and methacrylates and their quaternary salts, unsaturated nitriles, N-substituted maleimides, dienes, vinyl halides and vinylidene halides, vinyl esters, trialkylsilyl methacrylates and trialkoxysilyl methacrylates, 9-vinylcarbazole, vinyl-2-pyridine, vinyl-4-pyridine, 1-vinyl-2-pyrrolidinone, vinyltrimethoxysilane, vinyltrimethylsilane and 1-vinylimidazole.

8. Process according to one of Claims 5 to 7, characterized in that it produces an oligomer or a polymer in which n is an integer such that the number-average molecular mass of the sequence derived from Mv is between $10^2$ and $10^6$.

## Patentansprüche

### Patentansprüche für folgende Vertragsstaaten : AT, BE, CH, DE, DK, FR, GB, GR, IT, LI, LU, NL, SE

1. Funktionelle Thiuram-Disulfide, entsprechend folgender Formel:

(Ia) oder : (Ib)

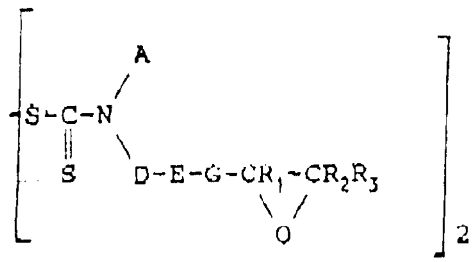

(Ia)

bzw. : (Ic)

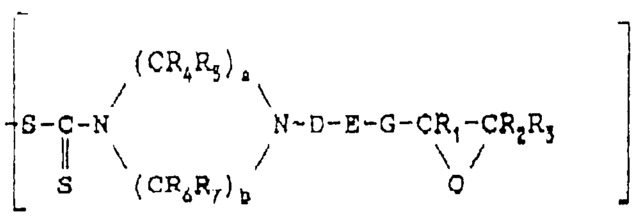

(Ib)

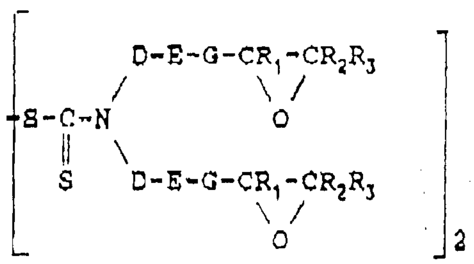

(Ic)

Hierbei gilt folgendes :

- A wird aus den Alkyl-, Cycloalkyl-, Aryl-, Arylalkyl-, Alkylaryl-Gruppen und den Polyoxyalkylen-Sequenzen ausgewählt, die durch folgende Formeln vertreten werden:

$$R_{10}\text{-O-(-}CH_2\text{-}CH_2\text{-O-)}_c\text{-}CH_2\text{-}CH_2\text{-}$$

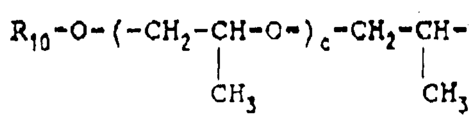

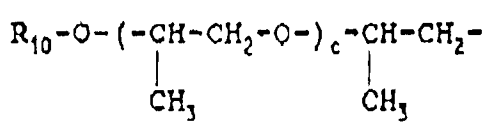

$$R_{10}\text{-O-[-}(CH_2)_4\text{-O-]}_c\text{-}(CH_2)_4\text{-}$$

Hierbei :

- vertritt $R_{10}$ einen Alkyl-, Cycloalkyl- oder Aryl-Rest; und :
- c hat Werte von 1 bis 1.000.

Darüberhinaus erfolgt die Auswahl aus jenen Polyester-Sequenzen, die durch folgende Formeln vertreten werden:

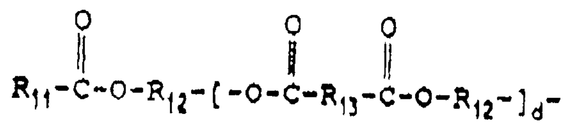

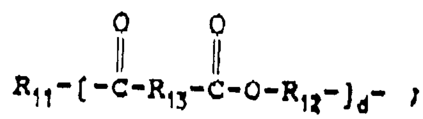

et

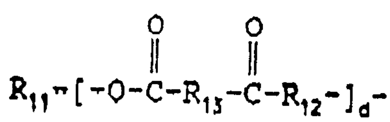

Hierbei gilt folgendes :

- $R_{11}$ entspricht den vorstehend für $R_{10}$ vorgenommenen Angaben;
- $R_{12}$ und $R_{13}$ werden aus den Alkylen-, Cycloalkylen-, Arylen-, Alcenylen- und Alcenylarylen-Gruppen ausgewählt und können mindestens ein Atom andersartiger Beschaffenheit innerhalb der Kette und / oder des Zyklus und/oder mindestens einen

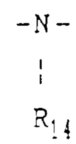

Rest umfassen; $R_{14}$ wird aus den Alkyl-, Cycloalkyl- und Aryl-Gruppen ausgewählt und kann Substituentien umfassen;
- d erhält Werte von 1 bis 300;
- D und G werden aus den Alkylen-, Cycloalkylen-, Arylen-, Alkylarylen- und Arylalkylen-Gruppen ausgewählt und können mindestens ein Atom andersartiger Beschaffenheit innerhalb der Kette und / oder des Zyklus umfassen;
- E vertritt folgendes :
  . einen Rest vom Typ

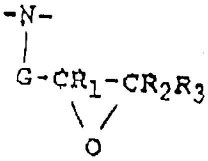

oder aber :

. einen -J-K-Rest, wobei J unter folgenden Gruppierungen ausgewählt wird

$CR_8R_9O$, 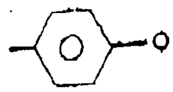 und 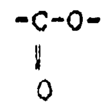

und :

. K vertritt eine einfache Bindung; oder aber es gilt folgendes (und zwar im Falle der Verbindungen vom Typ Ia) : J kann das Sauerstoffatom vertreten, und K kann folgenden Rest vertreten : (a) oder (b)

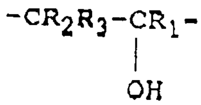 (a), oder 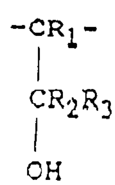 (b),

- $R_1$, $R_2$, $R_3$, $R_8$ und $R_9$ werden aus folgender Reihe ausgewählt : aus dem Wasserstoffatom und den Alkyl-, Cycloalkyl-, Aryl-, Alkylaryl- und Arylalkyl-Resten;
- $R_4$, $R_5$, $R_6$ und $R_7$ werden aus folgender Reihe ausgewählt: dem Wasserstoffatom und den Alkyl-Resten;
- a und b werden aus der Reihe der Ganzzahlen von 1 bis 3 ausgewählt.

2. Verfahren zur Herstellung der Verbindungen, die durch die Formel (Ia) oder (Ib) bzw. (Ic) vertreten werden, wobei diese Formel der Festlegung unter Anspruch 1 entspricht, und wobei E einen Rest vom Typ

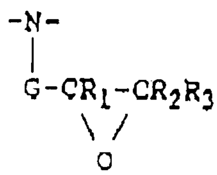

vertritt, dadurch gekennzeichnet, daß :

- man im Rahmen einer ersten Etappe eine Verbindung, die folgenden Formeln entspricht

$$A\text{-}NH\text{-}D\text{-}NH_2 \qquad (IIa)$$

oder :

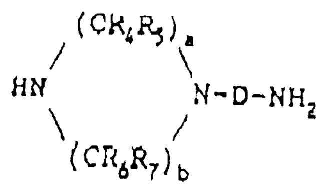 (IIb)

oder :

$$H_2N\text{-}D\text{-}NH\text{-}D\text{-}NH_2 \qquad (IIc)$$

mit einer Carbonyl-Verbindung reagieren läßt, die folgender Formel entspricht :

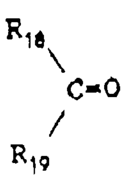

Hierbei entsprechen $R_{18}$ und $R_{19}$ der Festlegung, die vorstehend für $R_1$ vorgenommen wurde, unter Einhaltung der Bedingung, daß sie nicht beide dem Wasserstoffatom entsprechen dürfen, um die Verbindung zu erhalten, die folgender Formel entspricht :

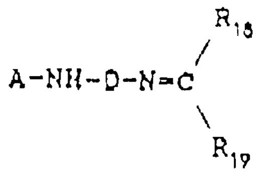

(IVa)

oder :

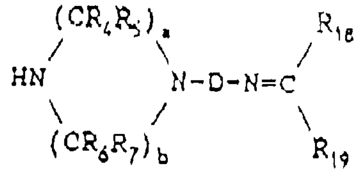

(IVb)

oder :

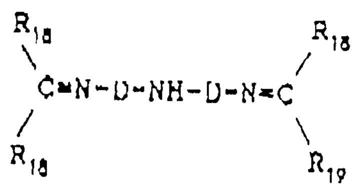

(IVc) ,

- Im Rahmen einer zweiten Phase läßt man die Verbindung (IVa) oder (IVb) oder (IVc) mit dem Kohlenstoff-Disulfid reagieren, in Anwesenheit mindestens eines Oxydationsmittels, um folgende Verbindung zu erhalten:

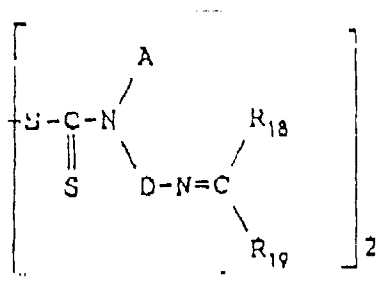

(Va)

oder :

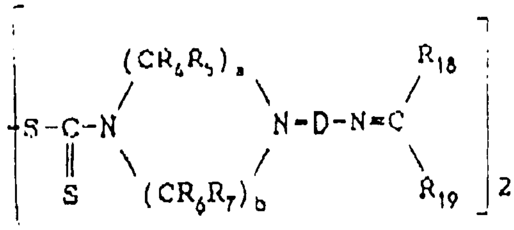

(Vb)

oder :

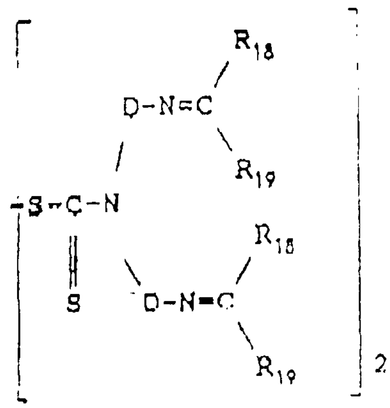

(Vc) ,

- Im Rahmen einer dritten Phase führt man eine Hydrolyse oder Reduktion der Verbindung (Va) oder (Vb) oder (Vc) vor, um eine Verbindung zu erhalten, die folgender Formel entspricht :

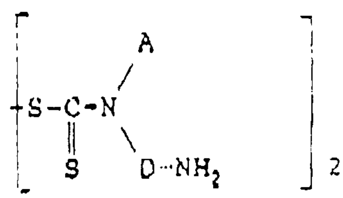

(VIa)

oder :

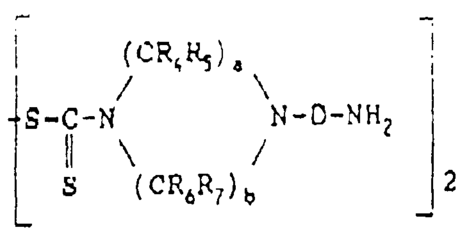

(VIb)

oder :

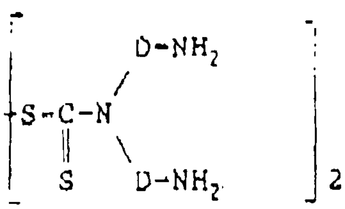

(VIc)

- Anschließend läßt man in einer vierten Phase die Verbindung (VIa) oder (VIb) oder (VIc) in einer basischen Umgebung mit einem Epoxyd reagieren, das folgender Formel entspricht :

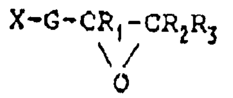

(VII)

hierbei vertritt X ein Halogen-Atom, und das Molverhältnis der Verbindung (VII) zur Verbindung (VIa) oder (VIb) oder (VIc) liegt bei mindestens 2.

**3.** Verfahren zur Herstellung von Verbindungen, die durch die Formel (Ia) oder (Ib) oder (Ic) vertreten werden, entsprechend der in Anspruch 1 gegebenen Definition; hierbei vertritt E jeweils -J-K-, und K vertritt eine einfache Bindung; dadurch gekennzeichnet, daß :

- man im Rahmen einer ersten Phase eine Verbindung, die folgender Formel entspricht :

A-NH-D-JH (VIIIa) oder :

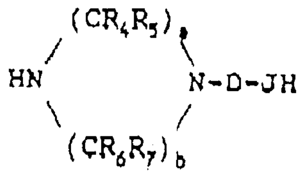

(VIIIb)

oder:

HJ-D-NH-D-JH (VIIIc)

- oder aber eines der quaternären Ammoniumsalze dieser Verbindung - mit dem Kohlenstoff-Disulfid reagieren läßt, in Anwesenheit mindestens eines Oxydationsmittels, um folgende Verbindung zu erhalten :

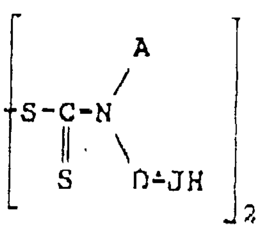

(IXa)

oder :

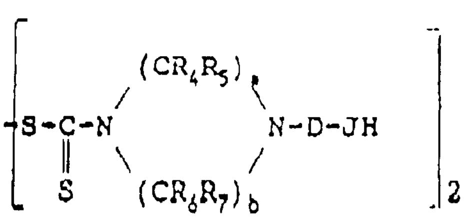

(IXb)

oder :

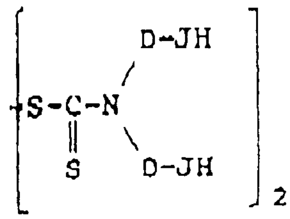

(IXc)

- Anschließend läßt man in einer zweiten Phase die Verbindung (IXa) oder (IXb) oder (IXc) in einer basischen Umgebung mit einem Mol-Überschuß des Epoxyds reagieren, das folgender Formel entspricht (VII):

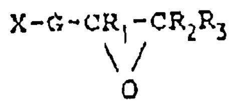

(VII)

Hierbei vertritt X ein Halogen-Atom.

4. Verfahren zur Herstellung von Verbindungen, die durch die Formel (Ia) oder (Ic) vertreten werden, wobei diese Formel jener Festlegung entspricht, die in Anspruch 1 gegeben worden ist, und wobei E jeweils -J-K- vertritt und K einen Rest des folgenden Typs vertritt:

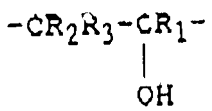

ou

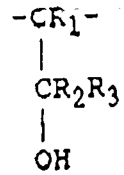

,

dadurch gekennzeichnet, daß :

- man im Rahmen einer ersten Phase eine Verbindung der Formel (VIIIa) oder (VIIIc) mit einer Halo-Säure HX reagieren läßt, um ein Salz zu erhalten, das folgenden Formeln entspricht :

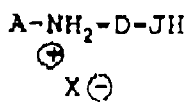

(Xa)

oder :

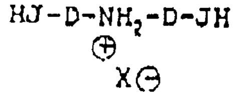

(Xc)

- und daß man im Rahmen einer zweiten Phase das Salz (Xa) oder (Xc) in basischer Umgebung mit einem Diepoxyd reagieren läßt, das folgender Formel entspricht :

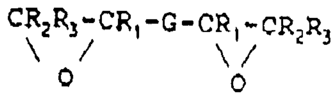

(XI)

um eine statistische Mischung von Verbindungen zu erhalten, die folgenden Formeln entsprechen :

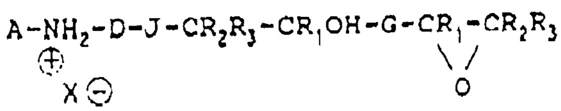

(XIIa)

und :

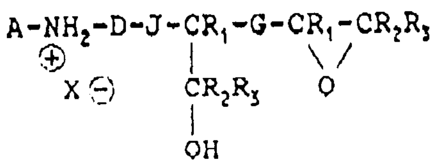

(XIIIa)

oder aber :

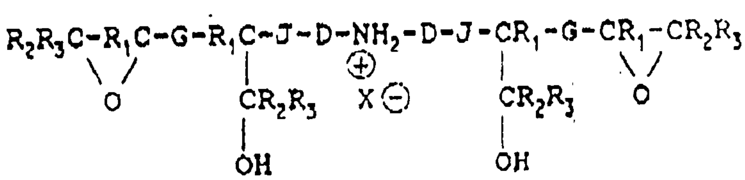

(XIIIc)

und :

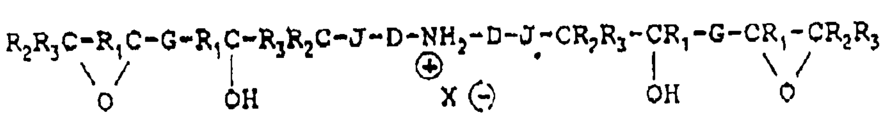

(XIIc) ;

- wobei man im Rahmen einer dritten Phase anschließend die Verbindungen (XIIa) und (XIIIa) oder aber (XIIc) und (XIIIc) mit dem Kohlenstoff-Disulfid reagieren läßt, in Anwesenheit mindestens eines Oxydationsmittels.

5. Verfahren zur Herstellung von Verbindungen, die durch die Formel (Ia) oder (Ic) vertreten werden, wie sie in Anspruch 1 festgelegt worden ist, wobei J das Sauerstoffatom vertritt, und wobei K entweder eine einfache Bindung oder aber einen Rest des folgenden Typs vertritt :

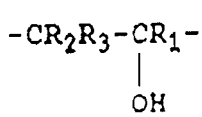

oder

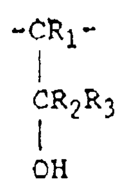

dadurch gekennzeichnet, daß man ein Thiuram-Disulfid, das folgender Formel entspricht :

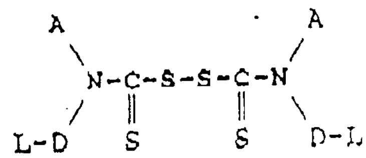

(XVa)

oder aber :

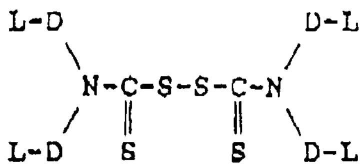

(XVc) ,

- wobei L eine Hydroxyl- oder aber ein $NHR_{20}$-Gruppe bezeichnet, und wobei $R_{20}$ jenen Festlegungen entspricht, die für $R_4$ gegeben worden sind - mit einem Epoxyd reagieren läßt, das folgender Formel entspricht :

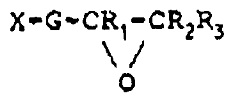

(VII)

bei der X ein Halogen-Atom vertritt;
oder aber die entsprechende Reaktion erfolgt mit einem Diepoxyd gemäß folgender Formel :

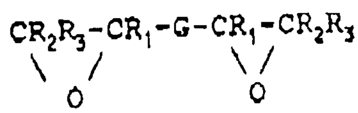

XI)

6. Einsatz eines funktionellen Thiuram-Disulfids - wie in Anspruch 1 festgelegt worden - , und zwar als ein Wirkstoff, der die dreifache Funktion eines Initiators, eines Hilfsmittels für die Verlagerung der Kette und schließlich eines Terminal-Agens erfüllt, im Rahmen der Radikal-Polymerisierung von Vinyl-Monomeren.

7. Oligomere und Polymere des Vinyl-Monomers Mv, mit folgender Formel :

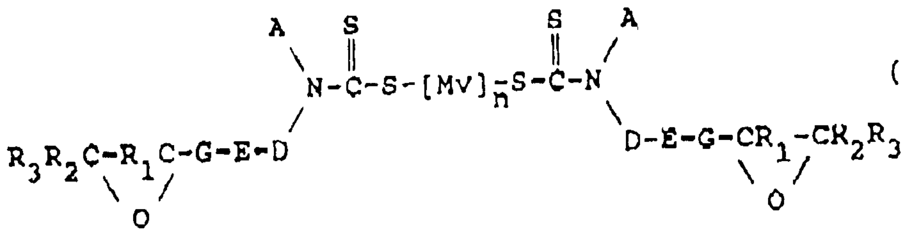

(XIVa),

oder aber

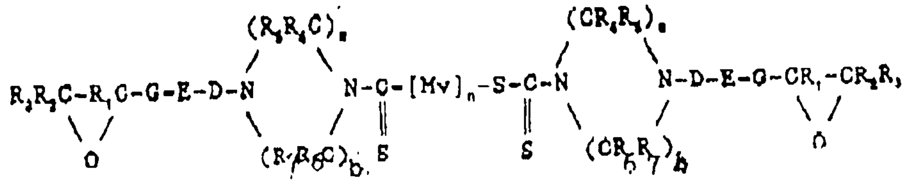

(XIVb)

oder aber

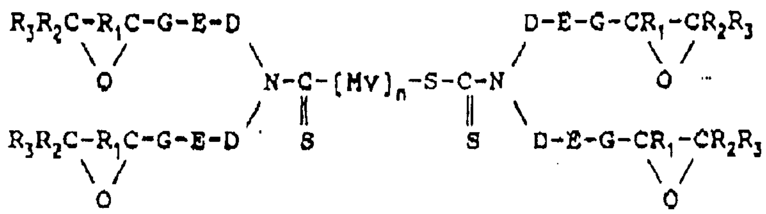

(XIVc)

Hierbei gilt folgendes :

- D, G, E, $R_1$, $R_2$, $R_3$, $R_4$, $R_5$, $R_6$ und $R_7$ - sowie a und b - entsprechen den Definitionen unter Anspruch 1;
- n ist eine ganze Zahl, die so zu wählen ist, daß die zahlenmittlere Molmasse der aus MV abgeleiteten Sequenz zwischen $10^2$ und $10^6$ liegt.

**8.** Oligomere und Polymere gemäß Anspruch 7, dadurch gekennzeichnet, daß Mv aus den Alkyl-Acrylaten und -Methacrylaten, wobei die entsprechende Alkyl-Gruppe gegebenenfalls mindestens ein andersartiges Atom umfaßt, und zwar in Form eines Halogen-Atoms, einer Carboxyl-Gruppe oder einer Amino-Gruppe, bzw aus den Aryl-Acrylaten und -Methacrylaten, den vinyl-aromatischen Kohlenwasserstoffen, den Epoxyd-Acrylaten und Methacrylaten, den Acrylamiden und Methacrylamiden, den Dialkylaminoalkyl-Acrylaten und Methacrylaten und ihren quaternären Salzen, den ungesättigten Nitrilen, den N-substituierten Maleimiden, den Dienen, den Vinyl- und Vinyliden-Halogeniden, den Vinyl-Estern, den Trialkylsilyl- und Trialcoxysilyl-Methacrylaten, dem 9-Vinyl-Carbazol, dem Vinyl-2-Pyridin, dem Vinyl-4-Pyridin, dem 1-Vinyl-2-Pyrrolidinon, dem Vinyltrimethoxysilan, dem Vinyltrimethylsilan und dem 1-Vinylimidazol ausgewählt wird.

**9.** Einsatz gemäß Anspruch 6, dadurch gekennzeichnet, daß die Menge des eingegebenen Iniferters zwischen 5 x $10^{-5}$ Mol pro Liter und 0,1 Mol pro Liter im Verhältnis zum Vinyl-Monomer liegt.

**10.** Oligomere und Polymere gemäß Anspruch 7, dadurch gekennzeichnet, daß n eine ganze Zahl ist, aufgrund derer die zahlenmittlere Molmasse der aus Mv abgeleiteten Sequenz zwischen $10^2$ und $10^6$ liegt.

**Patentansprüche für folgenden Vertragsstaat : ES**

**1.** Verfahren zur Herstellung von funktionellen Thiuram-Disulfiden, entsprechend den Formeln (Ia) oder (Ib) oder (Ic):

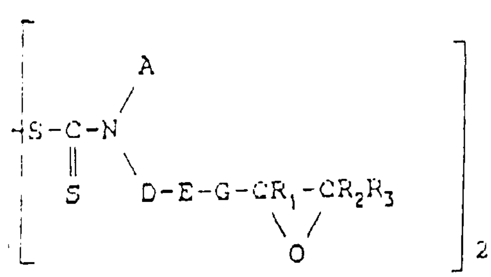

(Ia)

oder

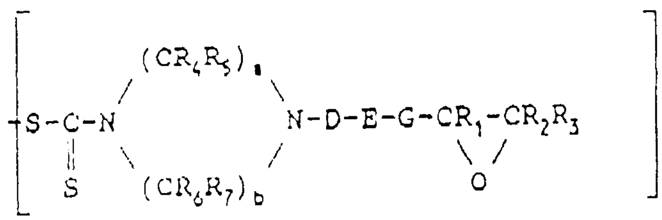

(Ib)

bzw. :

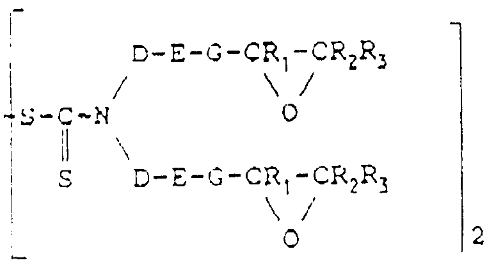

(Ic)

Hierbei gilt folgendes :

- A wird aus den Alkyl-, Cycloalkyl-, Aryl-, Arylalkyl-, Alkylaryl-Gruppen sowie aus den Polyoxyalkylen-Sequenzen ausgewählt, die durch folgende Formeln vertreten werden:

$$R_{10}\text{-O-(-CH}_2\text{-CH}_2\text{-O-)}_c\text{-CH}_2\text{-CH}_2\text{-}$$

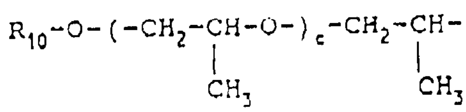

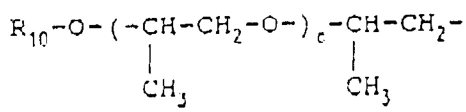

$$R_{10}\text{-O-[-CH}_2)_4\text{-O-]}_c\text{-(CH}_2)_4\text{-}$$

Hierbei :

- vertritt $R_{10}$ einen Alkyl-, Cycloalkyl- oder Aryl-Rest; und :
- c nimmt Werte von 1 bis 1.000 an.

Darüberhinaus erfolgt die Auswahl aus jenen Polyester-Sequenzen, die durch folgende Formeln vertreten werden:

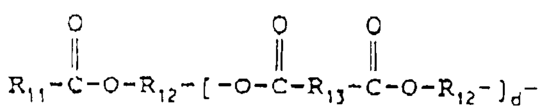

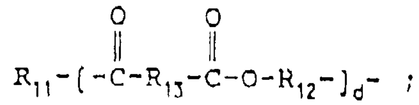

et

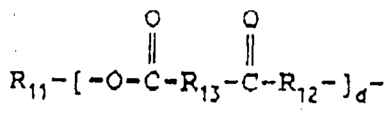

Hierbei gilt folgendes :

- $R_{11}$ entspricht den vorstehend für $R_{10}$ vorgenommenen Angaben;
- $R_{12}$ und $R_{13}$ werden aus den Alkylen-, Cycloalkylen-, Arylen-, Alcenylen- und Alcenylarylen-Gruppen ausgewählt und können mindestens ein Atom andersartiger Beschaffenheit innerhalb der Kette und / oder des Zyklus und/oder mindestens einen

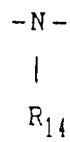

Rest umfassen; $R_{14}$ wird aus den Alkyl-, Cycloalkyl- und Aryl-Gruppen ausgewählt und kann Substituentien umfassen;
- d hat Werte von 1 bis 300;
- D und G werden aus den Alkylen-, Cycloalkylen-, Arylen-, Alkylarylen- und Arylalkylen-Gruppen ausgewählt und können mindestens ein Atom andersartiger Beschaffenheit innerhalb der Kette und / oder des Zyklus umfassen;
- E vertritt folgendes :

  . einen Rest vom Typ

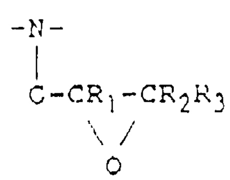

- $R_1$, $R_2$ und $R_3$ werden aus folgender Menge ausgewählt: aus dem Wasserstoffatom und den Alkyl-, Cycloalkyl-, Aryl-, Alkylaryl- und Arylalkyl-Resten;
- $R_4$, $R_5$, $R_5$ und $R_7$ werden aus folgender Menge ausgewählt : aus dem Wasserstoffatom und den Alkyl-Resten;
- a und b werden aus der Reihe der Ganzzahlen von 1 bis 3 ausgewählt,

  dadurch gekennzeichnet, daß :
- man im Rahmen einer ersten Phase eine Verbindung, die folgenden Formeln entspricht :

$$A\text{-}NH\text{-}D\text{-}NH_2 \qquad \text{(IIa) oder :}$$

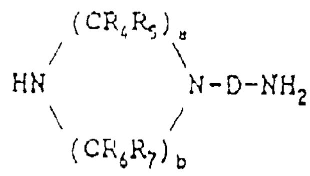 (IIb)

oder :

$H_2N\text{-}D\text{-}NH\text{-}D\text{-}NH_2$ (IIc)

mit einer Carbonyl-Verbindung reagieren läßt, die folgender Formel entspricht

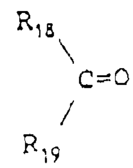

Hierbei entsprechen $R_{18}$ und $R_{19}$ der Festlegung, die vorstehend für $R_1$ vorgenommen wurde, unter Einhaltung der Bedingung, daß sie nicht beide dem Wasserstoffatom entsprechen dürfen, um die Verbindung zu erhalten, die folgender Formel entspricht :

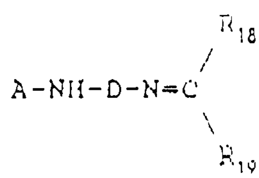 (IVa)

oder :

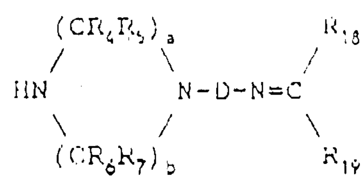 (IVb)

oder :

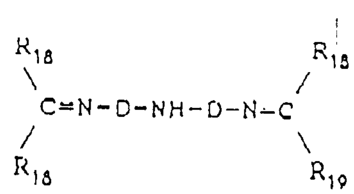 (IVc),

- im Rahmen einer zweiten Phase läßt man die Verbindung (IVa) oder (IVb) oder (IVc) mit dem Kohlenstoff-Disulfid reagieren, in Anwesenheit mindestens eines Oxydationsmittels, um folgende Verbindung zu erhalten:

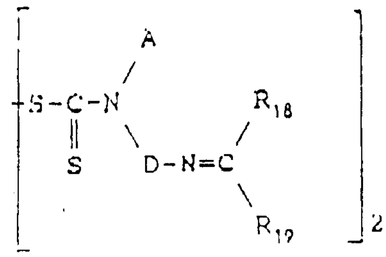

(Va)

oder :

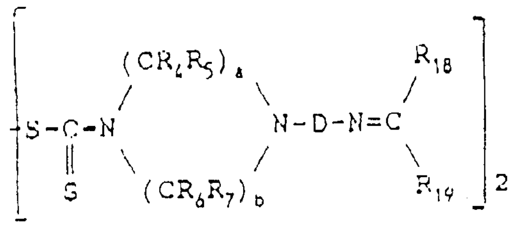

(Vb)

oder

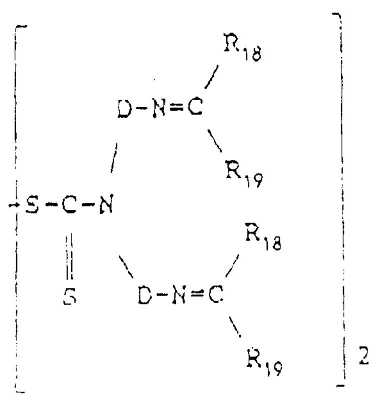

(Vc),

- Im Rahmen einer dritten Phase führt man eine Hydrolyse oder Reduktion der Verbindung (Va) oder (Vb) oder (Vc) vor, um eine Verbindung zu erhalten, die folgender Formel entspricht :

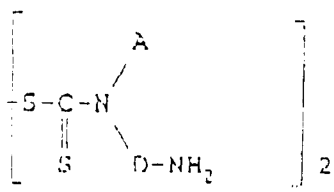

(VIa)

oder :

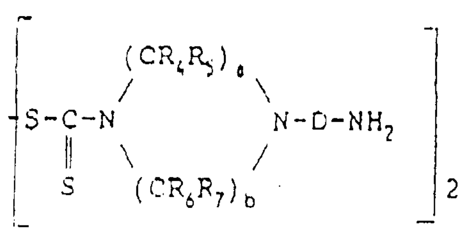

(VIb)

oder :

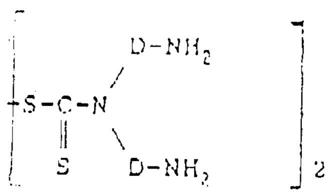

(VIc)

- Anschießend läßt man in einer vierten Phase die Verbindung (VIa) oder (VIb) oder (VIc) in einer basischen Umgebung mit einem Epoxyd reagieren, das folgender Formel entspricht :

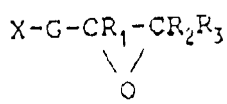

(VII)

hierbei vertritt X ein Halogen-Atom, und das Molverhältnis zwischen der Verbindung (VII) und der Verbindung (VIa) oder (VIb) oder (VIc) liegt bei mindestens 2.

**2.** Verfahren zur Herstellung von Thiuram-Disulfiden, die durch die Formel (Ia) oder (Ib) oder (Ic) vertreten werden; hierbei gilt folgendes :
- A, D, G, $R_1$ bis $R_7$ sowie a und b entsprechen den Festlegungen in Anspruch 1; und
- E vertritt einen -J-K-Rest; J wird aus den $CR_8R_9O$-Gruppierungen ausgewählt; $R_8$ und $R_9$ werden wie folgt ausgewählt : entweder als das Wasserstoffatom, oder aus den Alkyl-, Cycloalkyl-, Aryl-, Alkylaryl- und Arylalkyl-Resten der Formel

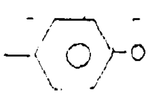 oder 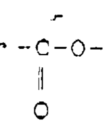

K vertritt schließlich eine einfache Bindung;
dadurch gekennzeichnet, daß :
- man im Rahmen einer ersten Phase eine Verbindung, die folgender Formel entspricht :

A-NH-D-JH (VIIIa) oder :

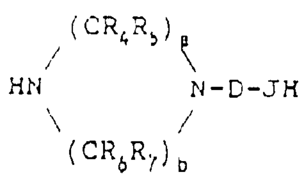

(VIIIb)

oder :

HJ-D-NH-D-JH (VIIIc)

- oder aber eines der quaternären Ammoniumsalze dieser Verbindungen - mit dem Kohlenstoff-Disulfid reagieren läßt, in Anwesenheit mindestens eines Oxydationsmittels, um folgende Verbindung zu erhalten :

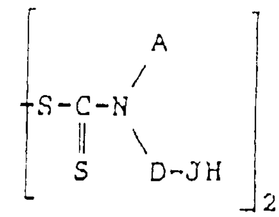

(IXa)

oder :

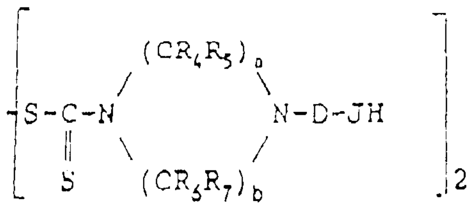

(IXb)

oder :

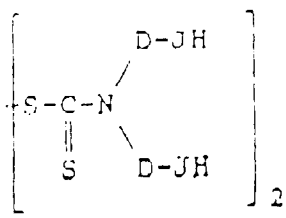

(IXc)

- Anschließend läßt man in einer zweiten Phase die Verbindung (IXa) oder (IXb) oder (IXc) in einer basischen Umgebung mit einem Mol-Überschuß des Epoxyds reagieren, das folgender Formel entspricht (VII):

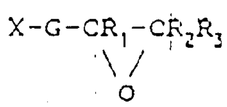

(VII)

Hierbei vertritt X ein Halogen-Atom.

3. Verfahren zur Herstellung von Thiuram-Disulfiden, die durch die Formel (Ia) oder (Ic) vertreten werden, wobei folgendes gilt :
   - A, D, G, $R_1$ bis $R_7$ sowie a und b entsprechen den Festlegungen in Anspruch 1; und :
   - E vertritt -J-K-;
   - J vertritt $CR_8R_9O$;
   - $R_8$ und $R_9$ entsprechen den Festlegungen unter Anspruch 2;

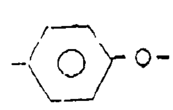 , 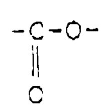

sowie K vertreten schließlich einen Rest des Typs :

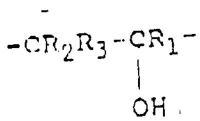

ou 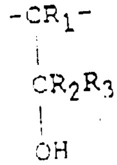 ,

dadurch gekennzeichnet, daß :

- man im Rahmen einer ersten Phase eine Verbindung der Formel (VIIIa) oder (VIIIc) mit einer Halo-Säure HX reagieren läßt, um ein Salz zu erhalten, das folgenden Formeln entspricht :

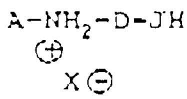

(Xa)

oder :

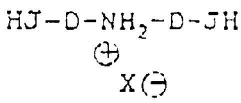

(Xc)

- und daß man im Rahmen einer zweiten Phase das Salz (Xa) oder (Xc) in basischer Umgebung mit einem Diepoxyd reagieren läßt, das folgender Formel entspricht :

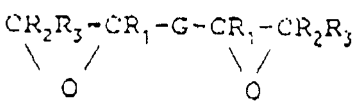

(XI)

um eine statistische Mischung der Verbindungen zu erhalten, die folgenden Formeln entsprechen :

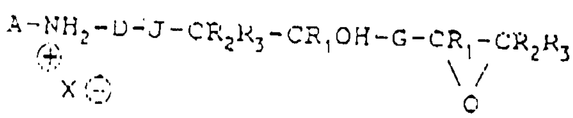

(XIIa)

und :

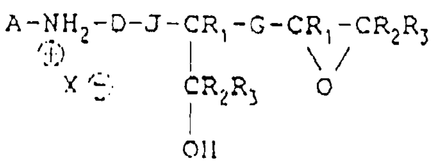

(XIIIa)

oder aber :

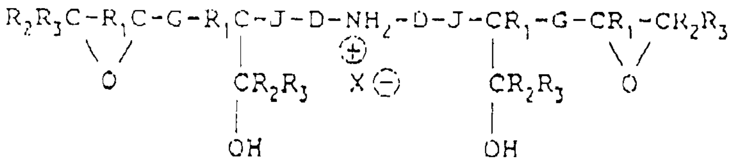 (XIIIc)

und :

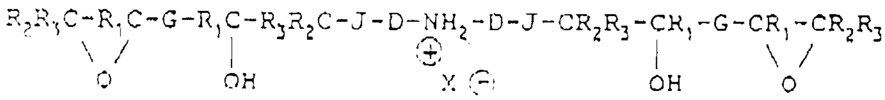 (XIIc) ;

- und wobei man im Rahmen einer dritten Phase anschließend die Verbindungen (XIIa) und (XIIIa) oder aber (XIIc) und (XIIIc) mit dem Kohlenstoff-Disulfid reagieren läßt, in Anwesenheit mindestens eines Oxydationsmittels.

**4.** Verfahren zur Herstellung von Thiuram-Disulfiden, die durch die Formeln (Ia) oder (Ic) vertreten werden, wobei folgendes gilt :
- A, D, G, $R_1$ bis $R_7$ sowie a und b entsprechen den Festlegungen in Anspruch 1; und :
- E vertritt -J-K-;
- J vertritt das Sauerstoffatom;
- K vertritt eine einfache Bindung oder aber einen Rest, der folgender Formel entspricht :

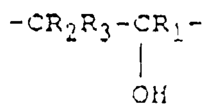

oder der aber folgender Form entspricht :

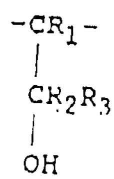

dadurch gekennzeichnet, daß man ein Thiuram-Disulfid, das folgender Formel entspricht :

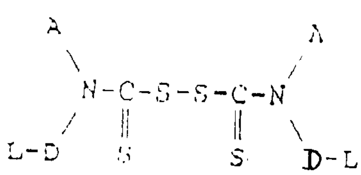 (XVa)

.

oder aber :

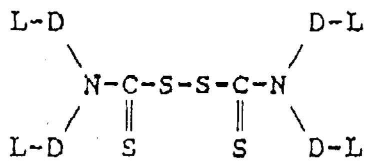

(XVc),

- wobei L eine Hydroxyl- oder aber ein $NHR_{20}$-Gruppe bezeichnet, und wobei $R_{20}$ jenen Festlegungen entspricht, die für $R_4$ gegeben worden sind - mit einem Epoxyd reagieren läßt, das folgender Formel entspricht :

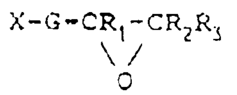

(VII)

bei der X ein Halogen-Atom vertritt;
oder aber die entsprechende Reaktion erfolgt mit einem Diepoxyd gemäß folgender Formel :

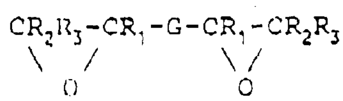

(XI)

**5.** Verfahren zur Radikal-Polymerisierung mindestens eines Vinyl-Monomers Mv, um Oligomere und Polymere des Vinyl-Monomers Mv zu erhalten, die folgenden Formeln entsprechen :

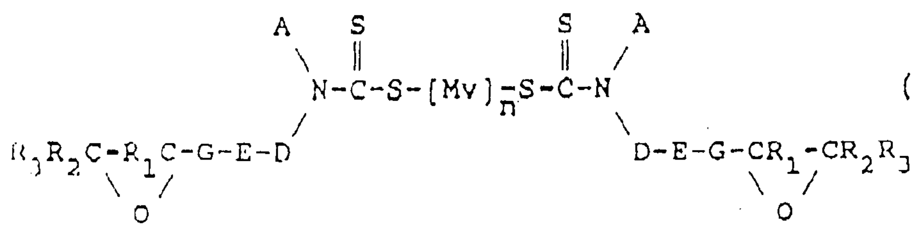

(XIVa)

oder aber :

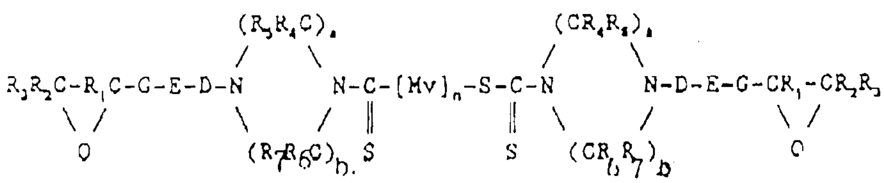

(XIVb)

oder aber :

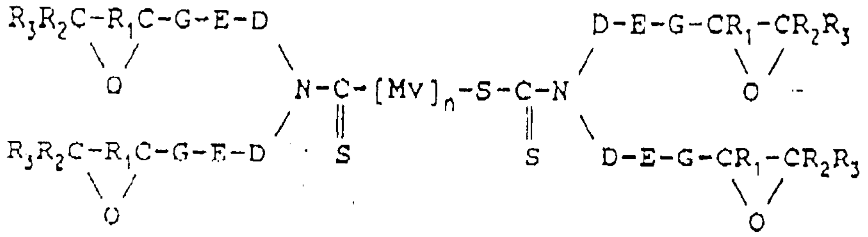

(XIVc)

Hierbei gilt folgendes :

- A, D, G, $R_1$, $R_2$, $R_3$, $R_4$, $R_5$, $R_6$ und $R_7$ - sowie a und b - entsprechen den Definitionen unter Anspruch 1;
- E entspricht den Festlegungen unter den Ansprüchen 1 und / oder 2 und / oder 3 und / oder 4 ; und :
- n ist eine ganze Zahl, die so zu wählen ist, daß die zahlenmittlere Molmasse der aus Mv abgeleiteten Sequenz zwischen $10^2$ und $10^6$ liegt,

dadurch gekennzeichnet, daß man mindestens ein funktionelles Thiuram-Disulfid, das mit einem Verfahren angefertigt wurde, wie es unter Anspruch 1 und / oder 2 und / oder 3 und / oder 4 festgelegt worden ist, einsetzt, und zwar als ein Wirkstoff, der die dreifache Funktion eines Initiators, eines Hilfsmittels für die Verlagerung der Kette und schließlich eines Terminal-Agens im Rahmen der Radikal-Polymerisierung von Vinyl-Monomeren erfüllt.

**6.** Verfahren gemäß Anspruch 5, dadurch gekennzeichnet, daß die Menge des eingegebenen Iniferters zwischen 5 x $10^{-5}$ Mol pro Liter und 0,1 Mol pro Liter - im Verhältnis zum Vinyl-Monomer - liegt.

**7.** Verfahren gemäß Anspruch 5, dadurch gekennzeichnet, daß Mv aus den Alkyl-Acrylaten und -Methacrylaten, deren Alkyl-Gruppe gegebenenfalls mindestens ein andersartiges Atom umfaßt, und zwar in Form eines Halogen-Atoms, einer Carboxyl-Gruppe oder einer Amino-Gruppe, bzw aus den Aryl-Acrylaten und -Methacrylaten, den vinyl-aromatischen Kohlenwasserstoffen, den Epoxyd-Acrylaten und Methacrylaten, den Acrylamiden und Methacrylamiden, den Dialkylaminoalkyl-Acrylaten und Methacrylaten und ihren quaternären Salzen, den ungesättigten Nitrilen, den N-substituierten Maleimiden, den Dienen, den Vinyl- und Vinyliden-Halogeniden, den Vinyl-Estern, den Trialkylsilyl- und Trialcoxysilyl-Methacrylaten, dem 9-Vinyl-Carbazol, dem Vinyl-2-Pyridin, dem Vinyl-4-Pyridin, dem 1-Vinyl-2-Pyrrolidinon, dem Vinyltrimethoxysilan, dem Vinyltrimethylsilan und dem 1-Vinylimidazol ausgewählt wird.

**8.** Verfahren gemäß einem der Ansprüche 5 bis 7, dadurch gekennzeichnet, daß das Verfahren zu einem Oligomer oder zu einem Polymer führt, bei dem n eine ganze Zahl ist, aufgrund derer die zahlenmittlere Molmasse der aus Mv abgeleiteten Sequenz zwischen $10^2$ und $10^6$ liegt.